(19) Europäisches Patentamt
European Patent Office
Office européen des brevets

(11) EP 1 054 657 B1

(12) EUROPÄISCHE PATENTSCHRIFT

(45) Veröffentlichungstag und Bekanntmachung des Hinweises auf die Patenterteilung:
**07.01.2004 Patentblatt 2004/02**

(21) Anmeldenummer: **99959302.3**

(22) Anmeldetag: **23.11.1999**

(51) Int Cl.[7]: **A61K 7/13**, A61K 7/135

(86) Internationale Anmeldenummer:
**PCT/EP1999/009005**

(87) Internationale Veröffentlichungsnummer:
**WO 2000/033799 (15.06.2000 Gazette 2000/24)**

# (54) MITTEL ZUR FÄRBUNG VON FASERN

AGENT FOR COLORING FIBERS

PRODUIT DE COLORATION DE FIBRES

(84) Benannte Vertragsstaaten:
**AT BE CH CY DE DK ES FI FR GB GR IE IT LI LU MC NL PT SE**

(30) Priorität: **07.12.1998 DE 19856342**
**12.05.1999 DE 29908464 U**
**16.07.1999 DE 19933313**
**21.07.1999 DE 19934283**

(43) Veröffentlichungstag der Anmeldung:
**29.11.2000 Patentblatt 2000/48**

(73) Patentinhaber: **Wella Aktiengesellschaft**
**64274 Darmstadt (DE)**

(72) Erfinder:
- **KUNZ, Manuela**
  **CH-1723 Marly (CH)**
- **MÜLLER, Catherine**
  **CH-1723 Marly (CH)**
- **OBERSON, Sylviane**
  **CH-1726 Farvagny (CH)**
- **UMBRICHT, Gisela**
  **CH-1723 Marly (CH)**
- **BRAUN, Hans-Jürgen**
  **CH-3182 Überstorf (CH)**
- **GÖTTEL, Otto**
  **CH-1723 Marly (CH)**
- **HAYOZ, André**
  **CH-1724 Senèdes (CH)**

(56) Entgegenhaltungen:
**EP-A- 0 370 492**
**EP-A- 0 376 776**
**EP-A- 0 497 697**
**EP-A- 0 826 668**
**US-A- 3 800 809**

EP 1 054 657 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Gegenstand der vorliegenden Erfindung ist ein Mittel zur Färbung von Fasern, insbesondere Keratinfasern (z.B. menschlichen Haaren), das aromatische Enamine und Carbonylverbindungen enthält, ein Verfahren zum Färben von Fasern, insbesondere Keratinfasern, ein Mehrkomponenten-Kit zur Färbung und späteren Entfärbung von Fasern, insbesondere von menschlichen Haaren, der sowohl Mittel zur Erzeugung einer Färbung auf der Faser als auch Mittel zur reduzierenden Entfernung der Färbung enthält, sowie ein Verfahren zum Färben und späteren Entfärben von Fasern, insbesondere Keratinfasern.

Haarfärbemittel werden je nach zu färbender Ausgangshaarfarbe und gewünschtem Endresultat hauptsächlich in den Bereich der Oxidationsfärbemittel oder der Tönungen unterteilt. Oxidationshaarfarben eignen sich hervorragend für die Abdeckung von höheren Grauanteilen, hierbei werden die bei einem Grauanteil von bis zu 50 % verwendeten Oxidationsfärbemittel in der Regel als oxidative Tönungen bezeichnet, während die bei einem Grauanteil von über 50 % oder zum "Hellerfärben" verwendeten Oxidationsfärbemittel in der Regel als sogenannte oxidative Farben bezeichnet werden. Direktziehende Farbstoffe sind hauptsächlich in nicht-oxidativen Färbemitteln (sogenannten Tönungsmitteln) enthalten. Einige direktziehende Farbstoffe wie z. B. die Nitrofarbstoffe, können aufgrund ihrer geringen Größe in das Haar eindringen und es - zumindestens in den äußeren Bereichen - direkt anfärben. Derartige Tönungen sind sehr haarschonend und überstehen in der Regel 6 bis 8 Haarwäschen und ermöglichen eine Grauabdeckung von etwa 20 %.

Im allgemeinen waschen sich direktziehende und oxidative Tönungen nach einigen Haarwäschen heraus. Die Zeitdauer hängt u. a. sehr stark von der Haarstruktur und der verwendeten Nuance ab. Oxidative Farben können teilweise mit der Zeit verblassen, verbleiben aber in der Regel bis zum nächsten Haarschnitt im Haar. Eine jederzeit mögliche Entfernung der Haarfärbung kann jedoch dann wünschenswert sein, wenn man eine besondere Farbe nur für einen bestimmten Zeitraum tragen will, oder eine Färbung dem Anwender nicht gefällt. Ebenso kann im Falle der Haarfärbung bei Erstverwendern die Möglichkeit einer schonenden und vollständigen Entfernung der Färbung die Angst vor einer zu drastischen Farbveränderung vermindern ("Färbung auf Probe").

Aus der DE-OS 197 45 292 ist die Verwendung einer Kombination von Malonaldehydderivaten, wie zum Beispiel Malonaldehyd-bis-dialkylacetalen, und Aminen oder CH-aciden Verbindungen zur Färbung von Haaren ohne Zusatz von Oxidationsmitteln bekannt. Ebenfalls ist es aus dem Stand der Technik, beispielsweise K.H.Schrader, "Grundlagen und Rezepturen der Kosmetika", 2.Auflage (1989), Seite 807+808, bekannt, sogenannte Abziehmittel, welche reduzierende oder oxidierende Wirkstoffe enthalten, zur Entfernung von mißlungenen Färbungen zu verwenden. Derartige Abziehmittel führen jedoch zu einer nicht unerheblichen Schädigung der Haare und ermöglichen nur in den seltensten Fällen eine vollständige Entfärbung.

Aus der EP-A-0 370 492 sind Indol-Aldehyd Haarfärbemittel bekannt.

Aufgabe der vorliegenden Erfindung ist es, ein Färbesystem zur Verfügung zu stellen, daß ohne den Zusatz von Oxidationsmitteln (wie zum Beispiel Wasserstoffperoxid) zum einen eine schonende, intensive und dauerhafte Färbung der Fasern und zum anderen eine schonende und vollständige Entfernung dieser Färbung zu jedem beliebigen Zeitpunkt ermöglicht.

Überraschenderweise wurde nunmehr gefunden, daß bei Verwendung eines durch Vermischen eines Enamins der Formel (I) mit einer Carbonylverbindung erhaltenen Färbemittels auf schonende Weise intensive Färbungen erzielt werden, welche zu einem beliebigen späteren Zeitpunkt wieder vollständig entfernt werden können.

Gegenstand der vorliegenden Erfindung ist daher ein Mittel zur Färbung von Fasern (A), wie zum Beispiel Wolle, Seide, Baumwolle oder Haaren und insbesondere menschlichen Haaren, welches durch Vermischen zweier Komponenten erhalten wird, und dadurch gekennzeichnet ist, daß die eine Komponente (Komponente A1 ) mindestens ein Enamin der Formel (I) oder dessen physiologisch verträgliches Salz,

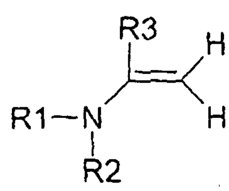

(I)

in der **R1** gleich einem ein- oder mehrkernigen aromatischen Rest, insbesondere einem gegebenenfalls mit einer C1- bis C4-Alkyfgruppe, C1- bis C4-Hydroxyalkylgruppe, Hydroxygruppe, Methoxygruppe, Dialkylaminogruppe oder Halogengruppe substituierten 5- gliedrigen oder 6-gliedrigen Arylrest, vorzugsweise einem Phenylrest, oder einem 5-gliedrigen oder 6-gliedrigen Heterozyklus, vorzugsweise einem Pyridylrest, oder einem Naphthylrest ist; **R2** gleich einer geradkettigen oder verzweigten C1- bis C8-Alkylgruppe, einer geradkettigen oder verzweigten C1- bis C8-Hydroxyalkylgruppe oder einer C1- bis C8-Alkoxyalkylgruppe, wobei zwischen den C-Atomen der Alkylkette Sauerstoffatome sitzen können, ist und **R3** gleich einer geradkettigen oder verzweigten C1- bis C8-Alkylgruppe, einer C1- bis C8-Alkoxyalkylgruppe, einer geradkettigen oder verzweigten C1- bis C8-Alkylengruppe, einer C1- bis C8-Alkoxyalkylengruppe, -O-, -NH-, -$NR_4$- oder -S- ist, mit $\mathbf{R_4}$ gleich einer Alkylgruppe, Alkoxyalkylgruppe, Hydroxyalkylgruppe oder Wasserstoff, wobei die Reste **R1** und **R3** gemeinsam mit dem Stickstoffatom und dem Kohlenstoffatom der Enamingrundstruktur eine zyklische Verbindung bilden können, enthält und die andere Komponente (Komponente A2) mindestens eine Carbonylverbindung, insbesondere einen Aldehyd, enthält.

Bevorzugt sind Verbindungen der Formel (I), in denen die Reste **R1** und **R3** gemeinsam mit dem Stickstoffatom und dem Kohlenstoffatom der Enamingrundstruktur eine zyklische Verbindung bilden, wobei vorzugsweise **R3** am aromatischen Rest **R1** mit dem Kohlenstoff verbunden ist, der in ortho-Stellung zum Enamin-substituierten Kohlenstoff steht.

Besonders bevorzugt sind Enamine der Formeln (II) bis (IX),

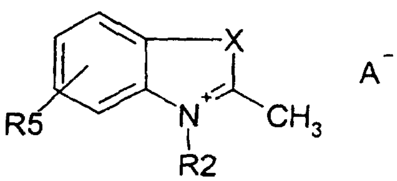
(II)

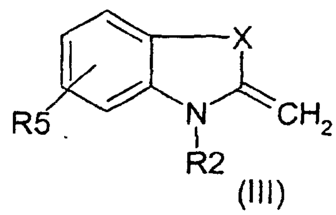
(III)

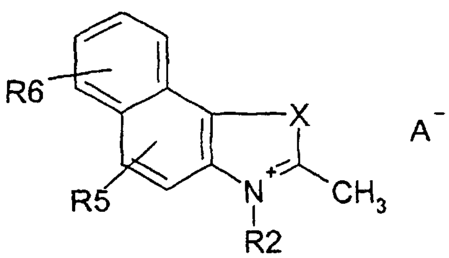
(IV)

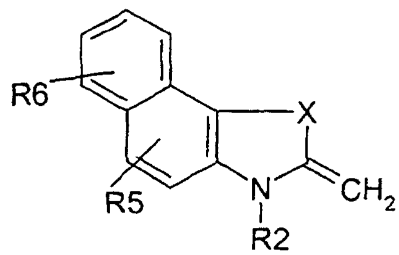
(V)

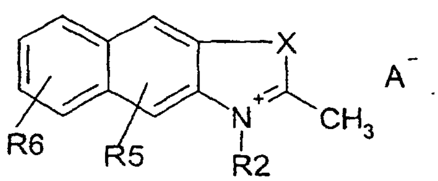
(VI)

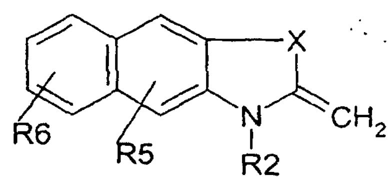
(VII)

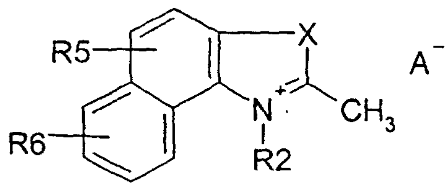

(VIII)

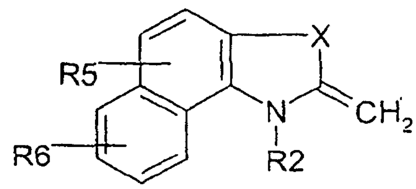

(IX)

in denen **X** gleich einem mit zwei C1- bis C4-Alkylgruppen (insbesondere 2 Methylgruppen) oder einer C1- bis C4-Alkylgruppe und einer Hydroxygruppe substituierten C-Atom, einem Schwefelatom, einem alkylierten oder nichtalkylierten Stickstoffatom, oder einem Sauerstoffatom ist; und **R2** gleich einer geradkettigen oder verzweigten C1- bis C8-Alkylgruppe, einer geradkettigen oder verzweigten C1- bis C8-Hydroxyalkylgruppe oder einer C1- bis C8-Alkoxyalkylgruppe ist, wobei zwischen den C-Atomen der Alkylkette Sauerstoffatome sitzen können; und **R5** und **R6** unabhängig voneinander gleich Wasserstoff, einer geradkettigen oder verzweigten C1- bis C4-Alkylgruppe, einer geradkettigen oder verzweigten C1- bis C4-Hydroxyalkylgruppe, einer Hydroxygruppe, einer Methoxygruppe, einer Aminogruppe, einer Dialkylaminogruppe oder einem Halogenatom sind; und **A**$^-$ gleich Chlorid, Bromid, Iodid, Hydrogensulfat, Monomethylsulfat, Sulfat, Hexafluor-phosphat, Hexafluorantimonat, Tetrafluorborat, Tetraphenylborat, vorzugsweise Chlorid, Bromid und Hydrogensulfat ist.

Unter den Verbindungen der Formeln (I) bis (IX) sind die folgenden Verbindungen besonders bevorzugt: 3-Ethyl-2-methylenbenzothiazolin, 2-Methylen-1,3,3-trimethyl-indolin, 1,2,3,3-Tetramethyl-3H-indoliniumchlorid, 1,2,3,3-Tetramethyl-3H-indolinium-bromid, 1,2,3,3-Tetramethyl-3H-indolinium-iodid, 1,2,3,3-Tetramethyl-3H-indoliniumsulfat, 1,2,3,3-Tetramethyl-3H-indolinium-hydrogensulfat, 1,2,3,3-Tetramethyl-3H-indolinium-methylsulfat, 1,2,3,3-Tetramethyl-3H-indoliniumhexafluorphosphat, 1,2,3,3-Tetramethyl-3H-indolinium-hexafluorantimonat, 1,2,3,3-Tetramethyl-3H-indolinium-tetrafluorborat, 5-Chloro-2-methylen-1,3,3-trimethyl-indolin oder dessen Salze, 1-(2-Hydroxyethyl)-3,3-dimethyl-2-methylen-indolin oder dessen Salze. 1,1,2,3-Tetramethyl-1H-benz[e]indolinium-chlorid, 1,1,2,3-Tetramethyl-1Hbenz[e]indolinium-bromid, 1,1,2,3-Tetramethyl-1H-benz[e]indolinium-iodid, 1,1,2,3-Tetramethyl-1H-benz[e]indolinium-sulfat, 1,1,2,3-Tetramethyl-1Hbenz[e]indolinium-hexafluorphosphat, 1,1,2,3-Tetramethyl-1H-benz[e]indolinium-methylsulfat, 1,1,2,3-Tetramethyl-1H-benz[e]indoliniumhexafluorantimonat und 1,1,2,3-Tetramethyl-1H-benz[e]indoliniumtetrafluorborat.

Als geeignete Carbonylverbindungen sind insbesondere die folgenden Aldehyde zu nennen: Vanillin (4-Hydroxy-3-methoxybenzaldehyd), Isovanillin (3-Hydroxy-4-methoxy-benzaldehyd), 3,4-Dihydroxybenzaldehyd, 4-Hydroxybenzaldehyd, 3,5-Dimethoxy-4-hydroxybenzaldehyd, 4-Dimethylaminobenzaldehyd, 4-Methyl-5-imidazolcarboxaldehyd, 4- Dimethylamino-zimtaldehyd, 4-Hydroxy-2-methoxybenzaldehyd, 3,5-Dimethyl-4-hydroxybenzaldehyd, 4-Dimethylamino-2-methoxybenzaldehyd, 2-Hydroxybenzaldehyd, 4-Hydroxy-1-naphthaldehyd, 4-Methoxy-1-naphthaldehyd, 4-Dimethylamino-1-naphthaldehyd, 4'-Hydroxy-biphenyl-1-carbaldehyd, 2-Hydroxy-3-methoxybenzaldehyd, 2,4-Dihydroxybenzaldehyd, 3,4-Dihydroxy-benzaldehyd, 2,5-Dihydroxybenzaldehyd, 2,3,4-Trihydroxybenzaldehyd, 3,4,5-Trihydroxybenzaldehyd, 2,4,6-Trihydroxybenzaldehyd, 2,4-Dimethoxybenzaldehyd, 2,3-Dimethoxybenzaldehyd, 2,5-Dimethoxy-benzaldehyd, 3,5-Dimethoxybenzaldehyd, 3,4-Dimethoxybenzaldehyd, Indol-3-carbaldehyd, Benzol-1,4-dicarbaldehyd, 4-Ethoxybenzaldehyd, 2-Methyl-1,4-naphthochinon, 4-Carboxybenzaldehyd, 4-Hydroxy-3-methoxyzimtaldehyd, 3,5-Dimethoxy-4-hydroxy-zimtaldehyd, 3-Methoxy-4-(1-pyrrofidinyl)-benzaldehyd, 4-Diethylamino-3-methoxybenzaldehyd, 1,2-Phthaldialdehyd, Pyrrol-2-aldehyd, Thiophen-2-aldehyd, Thiophen-3-aldehyd, Chromone-3-carboxaldehyd, 6-Methyl-4-oxo-1 (4H)-benzopyran-3-carbaldehyd, N-Methylpyrrol-2-aldehyd, 5-Methylfurfural, 6-Hydroxychromen-3-carboxaldehyd, 6-Methylindol-3-carboxaldehyd, 4-Dibutylaminobenzaldehyd, N-Ethylcarbazol-3-aldehyd, 4-Diethylamino-2-hydroxybenzaldehyd, 3,4-Dimethoxy-5-hydroxybenzaldehyd, 5-(4-(Diethylamino)phenyl)-2,4-pentadienal, 2,3-Thiophendicarboxaldehyd, 2,5-Thiophendicarboxaldehyd, 2-Methoxy-1-naphthaldehyd, 3-Ethoxy-4-hydroxybenzaldehyd, 2-Nitrobenzaldehyd, 3-Nitrobenzaldehyd und 4-Nitrobenzaldehyd.

Das Enamin und die Carbonylverbindung werden bis kurz vor der Anwendung voneinander getrennt aufbewahrt. Das erfindungsgemäße Färbemittel besteht in der Regel aus einer Mischung der beiden Komponenten A1 und A2, nämlich einer Farbträgermasse (A1), welche das Enamin und gegebenenfalls direktziehende Farbstoffe enthält, und einer weiteren Farbträgermasse (A2), welche die Carbonylverbindung und gegebenenfalls direktziehende Farbstoffe enthält. Diese beiden Komponenten werden unmittelbar vor der Anwendung zu einem gebrauchsfertigen Färbemittel vermischt und sodann auf die zu färbende Faser aufgetragen. Selbstverständlich ist es auch möglich. daß eine oder beide Komponenten aus mehreren Einzelkomponenten bestehen, welche vor der Anwendung miteinander

vermischt werden.

Die Enamine der Formel (I) und die Carbonylverbindungen sind in der jeweiligen Farbträgermasse (Komponente A1 bzw. Komponente A2) jeweils in einer Gesamtmenge von etwa 0,02 bis 20 Gewichtsprozent, vorzugsweise 0,2 bis 10 Gewichtsprozent, enthalten, wobei in dem durch Vermischen der Komponenten A1 und A2 erhaltenen gebrauchsfertigen Färbemittel A das Enamin der Formel (I) und die Carbonylverbindung jeweils in einer Gesamtmenge von etwa 0,01 bis 10 Gewichtsprozent. vorzugsweise 0,1 bis 5 Gewichtsprozent, enthalten ist.

Weiterhin kann das erfindungsgemäße Färbemittel gegebenenfalls zusätzlich übliche, physiologisch unbedenkliche, direktziehende Farbstoffe aus der Gruppe der Nitrofarbstoffe, Azofarbstoffe, Chinonfarbstoffe und Triphenylmethanfarbstoffe enthalten.

Die direktziehenden Farbstoffe können in der Komponente A1 und der Komponente A2 jeweils in einer Gesamtenge von etwa 0,02 bis 20 Gewichtsprozent, vorzugsweise 0,2 bis 10 Gewichtsprozent, eingesetzt werden , wobei die Gesamtmenge an direktziehenden Farbstoffen in dem durch Vermischen der Komponenten A1 und A2 erhaltenen gebrauchsfertigen Färbemittel A etwa 0,01 bis 10 Gewichtsprozent, vorzugsweise 0,1 bis 5 Gewichtsprozent, beträgt.

Die Zubereitungsform für das Färbemittel A und die Komponenten A1 und A2 kann beispielsweise eine Lösung, insbesondere eine wäßrige oder wäßrig-alkoholische Lösung sein. Weitere geeignete Zubereitungsformen sind eine Creme, ein Gel, ein Aerosolschaum oder eine Emulsion. Ihre Zusammensetzung stellt eine Mischung der Enamine der Formel (I) und/oder der Carbonylverbindungen mit den für solche Zubereitungen üblichen Zusätzen dar.

Übliche in Färbemitteln verwendete Zusätze in Lösungen, Cremes, Emulsionen, Gelen oder Aerdsolschäumen sind zum Beispiel Lösungsmittel wie Wasser, niedere aliphatische Alkohole, beispielsweise Ethanol, n-Propanol und Isopropanol oder Glykole wie Glycerin und 1,2-Propandiol, weiterhin Netzmittel oder Emulgatoren aus den Klassen der anionischen, kationischen, amphoteren oder nichtionogenen oberflächenaktiven Substanzen wie Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohle, oxethylierte Nonylphenole, Fettsäurealkanolamide, oxethylierte Fettalkohole, oxethylierte Nonylphenole, Fettsäurealkanolamide, oxethylierte Fettsäureester, ferner Verdicker wie höhere Fettalkohole, Stärke oder Cellulosederivate, Parfüme, Haarvorbehandlungsmittel, Konditionierer, Haarquellmittel, Konservierungsstoffe, weiterhin Vaseline, Paraffinöl und Fettsäuren sowie außerdem Pflegestoffe wie kationische Harze, Lanolinderivate, Cholesterin, Pantothensäure und Betain. Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent (bezogen auf die Farbträgermasse), die Verdicker in einer Menge von etwa 0,1 bis 25 Gewichtsprozent (bezogen auf die Farbträgermasse) und die Pflegestoffe in einer Konzentration von etwa 0,1 bis 5,0 Gewichtsprozent (bezogen auf die Farbträgermasse).

Der pH-Wert des gebrauchsfertigen Färbemittels A beträgt in der Regel 3 bis 11, vorzugsweise 6 bis 11, wobei sich der pH-Wert des gebrauchsfertigen Färbemittels bei der Mischung der vorzugsweise alkalisch eingestellten enamin-haltigen Komponente A1 mit der vorzugsweise sauer eingestellten carbonyl-haltigen Komponente A2 auf einen Wert einstellt, der durch die Alkalimenge in der Komponente A1 und die Säuremenge in der Komponente A2 sowie durch das Mischungsverhältnis dieser beiden Komponenten beinflußt wird.

Zur Einstellung des für die Färbung gewünschten pH-Wertes können alkalisierende Mittel wie Alkanolamine, Alkylamine, Alkalihydroxide oder Ammoniumhydroxid und Alkalicarbonate oder Ammoniumcarbonate oder Säuren wie Milchsäure, Essigsäure, Weinsäure, Phosphorsäure, Salzsäure, Zitronensäure, Ascorbinsäure und Borsäure, verwendet werden.

Das gebrauchsfertige Färbemittel A wird unmittelbar vor der Anwendung durch Vermischen der das Enamin enthaltenden Komponente A1 mit der die Carbonylverbindung enthaltenden Komponente A2 hergestellt und sodann auf die Faser aufgetragen. Je nach gewünschter Farbtiefe läßt man diese Mischung 5 bis 60 Minuten, vorzugsweise 15 bis 30 Minuten, bei einer Temperatur von 20 bis 50 Grad Celsius, insbesondere bei 30 bis 40 Grad Celsius einwirken. Anschließend wird die Faser mit Wasser gespült und gegebenenfalls mit einem Shampoo gewaschen.

Das erfindungsgemäße Färbemittel A ermöglicht eine schonende, gleichmäßige und dauerhafte Färbung der Fasem, insbesondere von Keratinfasern, wie zum Beispiel Haaren. Überraschenderweise können diese Färbungen zu einem beliebigen Zeitpunkt schnell und schonend durch Reduktionsmittel wieder vollständig entfärbt werden.

Ein weiter Gegenstand der vorliegenden Erfindung ist daher ein Mehrkomponenten-Kit zur Färbung und späteren Entfärbung von Fasern, wie zum Beispiel Wolle, Seide, Baumwolle oder Haaren und insbesondere menschlichen Haaren, welcher dadurch gekennzeichnet ist, daß er das erfindungsgemäße Färbemittel A und eine entfärbende Komponente B enthält, wobei die Komponente B als entfärbendes Agenz mindestens ein Sulfit, beispelsweise ein Ammoniumsulfit, Alkalisulfit oder Erdalkalisulfit, insbesondere Natriumsulfit oder Ammoniumsulfit, enthält.

Die Gesamtmenge an Sulfiten in der Komponente B beträgt etwa 0,1 bis 10 Gewichtsprozent, vorzugsweise 2 bis 5 Gewichtsprozent.

Das Mittel zur Entfärbung der dem Färbemittel A gefärbten Fasern (im folgenden "Entfärbemittel" genannt) kann als wäßrige oder wäßrig-alkoholische Lösung, als Gel, Creme, Emulsion oder Schaum vorliegen, wobei das Entfärbemittel sowohl in Form eines Einkomponentenpräparats als auch in Form eines Mehrkomponentenpräparates

konfektioniert sein kann. Das Entfärbemittel kann neben der Pulverform zum Schutz vor Staubbildung auch als Tablette - auch Brausetablette - oder Granulat konfektioniert sein. Hieraus wird dann vor der Anwendung mit kaltem oder warmem Wasser, gegebenenfalls unter Zusatz eines oder mehrerer der nachfolgend genannten Hilfsmittel, das Entfärbemittel hergestellt. Es ist jedoch auch möglich, daß diese Hilfsmittel (sofern sie in fester Form vorliegen) bereits in dem Entfärbepulver oder Entfärbegranulat beziehungsweise der Brausetablette enthalten sind. Durch Benetzung des Pulvers durch Öle oder Wachse kann zusätzlich die Staubbildung vermindert werden.

Das Entfärbemittel kann zusätzliche Hilfsmittel, wie zum Beispiel Lösungsmittel wie Wasser, niedere aliphatische Alkohole, beispielsweise Ethanol, n-Propanol und Isopropanol, Glykolether oder Glykole wie Glycerin und insbesondere 1,2-Propandiol, weiterhin Netzmittel oder Emulgatoren aus den Klassen der anionischen, kationischen, amphoteren oder nichtionogenen oberflächenaktiven Substanzen wie Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohle, oxethylierte Nonylphenole, Fettsäurealkanolamide, oxethylierte Fettalkohole, oxethylierte Nonylphenole, Fettsäurealkanolamide, oxethylierte Fettsäureester, ferner Verdicker wie höhere Fettalkohole, Stärke oder Cellulosederivate, Parfüme, Haarvorbehandlungsmittel, Konditionierer, Haarquellmittel, Konservierungsstoffe, Vaseline, Paraffinöl und Fettsäuren sowie außerdem Pflegestoffe wie kationische Harze, Lanolinderivate, Cholesterin, Pantothensäure und Betain, enthalten.

Der pH-Wert des Entfärbemittels beträgt etwa 3 bis 8, insbesondere 4 bis 7. Erforderlichenfalls kann der gewünschte pH-Wert durch Zugabe von geeigneten Säuren, beispielsweise $\alpha$-Hydroxycarbonsäuren wie Milchsäure, Weinsäure, Zitronensäure oder Äpfelsäure, Phosphorsäure, Essigsäure, Glycolsäure Salicylsäure, Glutathion oder Gluconsäurelacton, oder aber alkalisierenden Mitteln wie Alkanolaminen, Alkylaminen, Alkalihydroxiden, Ammoniumhydroxid, Alkalicarbonaten, Ammoniumcarbonaten oder Alkaliphosphaten, eingestellt werden.

Die Einwirkungszeit des Entfärbemittels beträgt je nach zu entfärbender Färbung und Temperatur (etwa 20 bis 50 Grad Celsius) 5 bis 60 Minuten, insbesondere 15 bis 30 Minuten, wobei durch Wärmezufuhr der Entfärbeprozeß beschleunigt werden kann. Nach Beendigung der Einwirkungszeit des Entfärbemittels wird das Haar mit Wasser gespült, gegebenenfalls mit einem Shampoo gewaschen.

Obwohl die Komponente B zur Entfärbung von mit dem Färbemittel A gefärbten Haaren, insbesondere menschlichen Haaren, besonders gut geeignet ist, kann die Komponente B auch zur Entfärbung von anderen mit dem Färbemittel A gefärbten natürlichen oder synthetischen Fasern, wie zum Beispiel Baumwolle, Wolle, Seide, Viskose, Nylon, Celluloseacetat, verwendet werden.

Die nachfolgenden Beispiele sollen den Gegenstand näher erläutern, ohne ihn auf diese Beispiele zu beschränken.

**Beispiele**

**Beispiele 1.1 bis 1.6: Haarfärbemittel**

| **Enaminhaltige Komponente A1** | |
|---|---|
| Enamin der Formel (I)<br>[mit $\alpha$-Tocopherol (Vitamin E) stabilisiert] | Mengenangaben<br>gemäß Tabelle 1 |
| Cetylstearylalkohol | 12,00 g |
| Stearylalkohol-polyethylenglykolether mit 20 Mol Ethylenoxid im Molekül (Steareth-20) | 1,40 g |
| Isopropanol | 20,0 g |
| Wasser, vollentsalzt | ad 100,0 g |
| **Aldehydhaltige Komponente A2** | |
| Aldehydverbindung | Mengenangaben gemäß Tabelle 1 |
| direktziehender Farbstoff | Mengenangaben gemäß Tabelle 1 |
| Cetylstearylalkohol | 3,06 g |
| Natriumlaurylsulfat | 0,34 g |
| Lanolinalkohol | 0,50 g |
| Wasser, vollentsalzt | ad 100,00 g |

5 g der Komponente A1 werden mit 5 g der Komponente A2 vermischt. Das erhaltene gebrauchsfertige Haarfärbemittel wird auf gebleichte Haare aufgetragen und mit einem Pinsel gleichmäßig verteilt. Nach einer Einwirkungszeit von 30 Minuten bei 40 °C wird das Haar mit einem Shampoo gewaschen, anschließend mit lauwarmem Wasser gespült und sodann getrocknet.

**Tabelle 1:** Färbe-Resultate

| **Nr.** | **a) Enaminhaltige Komponente A1**<br>**b) Aldehydhaltige Komponente A2** | **Farbton nach dem Färben** | | **Farbmeßwerte**<br>**L** | **a** | **b** |
|---|---|---|---|---|---|---|
| **1.1** | in (a) 1,3,3-Trimethyl-2-methylen-indolin 2,0 g | intensiv-rot | unbehandelte Haare: | +83,30; | -0,48; | +10,40 |
| | in (b) 4-Hydroxy-3-methoxy-benzaldehyd 1,76 g | | Nach dem Färben: | +25,32; | +58,19; | +12,67 |
| **1.2** | in a) 1,3,3-Trimethyl-2-methylen-indolin 2,0 g | intensiv-orange | unbehandelte Haare: | +83,30; | -0,48; | +10,40 |
| | in b) 4-Hydroxybenzaldehyd 1,43 g | | Nach dem Färben: | +44,34; | +73,22; | +42,70 |
| **1.3** | in a) 1,3,3-Trimethyl-2-methylen-indolin 2,0 g | intensiv-gelb | unbehandelte Haare: | +83,30; | -0,48; | +10,40 |
| | in b) 3-Hydroxy-4-methoxy-benzaldehyd 1,76 g | | Nach dem Färben: | +58,96; | +33,01; | +66,62 |

Tabelle 1: (Fortsetzung)

| Nr. | a) Enaminhaltige Komponente A1<br>b) Aldehydhaltige Komponente A2 | Farbton nach dem Färben | | Farbmeßwerte L | a | b |
|---|---|---|---|---|---|---|
| 1.4 | in a) 1,3,3-Trimethyl-2-methylen-indolin 2,0 g<br>in b) 4-Dimethylaminobenzaldehyd 1,72 g | intensiv-rosa | unbehandelte Haare:<br>Nach dem Färben: | +83,30;<br>+45,75; | -0,48;<br>+77,26; | +10,40<br>+0,54 |
| 1.5 | in a) 1,3,3-Trimethyl-2-methylen-indolin 2,0 g<br>in b) 3,4-Dihydroxybenzaldehyd 1,59 g | intensiv-weinrot | unbehandelte Haare:<br>Nach dem Färben: | +83,30;<br>+21,81; | -0,48;<br>+37,19; | +10,40<br>+5,12 |

Tabelle 1: (Fortsetzung)

| Nr. | a) Enaminhaltige Komponente A1<br>b) Aldehydhaltige Komponente A2 | Farbton nach dem Färben | | Farbmeßwerte L | a | b |
|---|---|---|---|---|---|---|
| 1.6 | in a) 1,3,3-Trimethyl-2-methylen-indolin 2,0 g<br>in b) 3,4-Dihydroxy-benzaldehyd 0,80 g<br>3-Hydroxy-4-methoxy-benzaldehyd 0,88 g<br>1-(2-Hydroxyethyl)amino-2-nitro-4-[di(2-hydroxyethyl)amino]-benzol (HC Blue No. 2) 2,00 g | intensiv-rotbraun | unbehandelte Haare:<br>Nach dem Färben: | +83,30;<br>+19,86; | -0,48;<br>+16,36; | +10,40<br>+3,51 |

**Beispiele 1.7 bis 1.21: Haarfärbemittel**

| Enamin der Formel (I) enthaltende Komponente A1 | |
|---|---|
| Enamin der Formel (I) | Mengenangaben gemäß Tabelle 1 |
| 6-O-Palmitoyl-L-ascorbinsäure | 0,30 g |
| Cetylstearylalkohol | 12,00 g |
| Laurylethersulfat 28 %ig | 10,00 g |
| Ethanol | 23,00 g |
| Wasser, vollentsalzt | ad 100,00 g |

Der Cetylstearylalkohol wird bei 80 °C geschmolzen. Das Laurylethersulfat wird mit 95 % des Wassers auf 80 °C erhitzt und zum geschmolzenen Cetylstearylalkohol gegeben und gerührt bis sich eine Creme ergibt. Bei Raumtemperatur wird Verbindung (I) mit dem restlichen Wasser und dem Alkohol und mit 6-O-Palmitoyl-L-ascorbinsäure versetzt und sodann zugegeben. Der pH-Wert der Creme wird mit 10 %iger wäßriger NaOH auf 12,0 eingestellt.

| Aldehydhaltige Komponente A2 | |
|---|---|
| Aldehydverbindung | Mengenangaben gemäß Tabelle 1 |
| direktziehender Farbstoff | Mengenangaben gemäß Tabelle 1 |
| Cetylstearylalkohol | 12,00 g |
| Laurylethersulfat 28 %ig | 10,00 g |
| Ethanol | 23,00 g |
| Wasser, vollentsalzt | ad 100,00 g |

Der Cetylstearylalkohol wird bei 80 °C geschmolzen. Das Laurylethersulfat wird mit 95 % des Wassers auf 80 °C erhitzt und zum geschmolzenen Cetylstearylalkohol gegeben und gerührt bis sich eine Creme ergibt. Bei Raumtemperatur wird die Aldehydverbindung sowie ggfs. die direktziehenden Farbstoffe, mit dem restlichen Wasser und dem Alkohol versetzt, zugegeben. Der pH-Wert der Creme wird mit 10 %iger wäßriger Milchsäure auf 4,0 eingestellt.

5 g der Komponente A1 werden mit 5 g der Komponente A2 vermischt. Das erhaltene gebrauchsfertige Haarfärbemittel wird auf eine Haarsträhne aufgetragen und mit einem Pinsel gleichmäßig verteilt. Nach einer Einwirkungszeit von 30 Minuten bei 40 °C wird das Haar mit einem Shampoo gewaschen, anschließend mit lauwarmem Wasser gespült und sodann getrocknet.

Die erhaltenen Färbungen sind in der nachfolgenden Tabelle 2 zusammengefaßt.

Tabelle 2: Färbe-Resultate

| Nr. | a) Enaminhaltige Komponente A1<br>b) Aldehydhaltige Komponente A2 | Farbton nach dem Färben | Farbmeßwerte | L | a | b |
|---|---|---|---|---|---|---|
| 1.7 | in (a) 1,1,2,3-Tetramethyl-1H-benz[e]indolinium-chlorid 2,99 g<br>in (b) 4-Hydroxy-3-methoxy-benzaldehyd 1,76 g | rosarot | unbehandelte Haare:<br>Nach dem Färben: | +83,30;<br>+47,43; | -0,48;<br>+50,09; | +10,40<br>+3,75 |
| 1.8 | in a) 1,1,2,3-Tetramethyl-1H-benz[e]indolinium-bromid 3,51 g<br>in b) 4-Hydroxybenzaldehyd 1,43 g | orange | unbehandelte Haare:<br>Nach dem Färben: | +83,30;<br>+60,27; | -0,48;<br>+53,71; | +10,40<br>+43,96 |
| 1.9 | in a) 1,1,2,3-Tetramethyl-1H-benz[e]indolinium-chlorid 2,99 g<br>in b) 3-Hydroxy-4-methoxy-benzaldehyd 1,76 g | gelb | unbehandelte Haare:<br>Nach dem Färben: | +83,30;<br>+68,20; | -0,48;<br>+22,52; | +10,40<br>+57,04 |

Tabelle 2: (Fortsetzung)

| Nr. | a) Enaminhaltige Komponente A1<br>b) Aldehydhaltige Komponente A2 | Farbton nach dem Färben | | Farbmeßwerte L | a | b |
|---|---|---|---|---|---|---|
| 1.10 | in a) 1,1,2,3-Tetramethyl-1H-benz[e]-indolinium-methylsulfat 3,86 g<br>in b) 4-Dimethylaminobenzaldehyd 1,72 g | rosa | unbehandelte Haare:<br>Nach dem Färben: | +83,30;<br>+52,30; | -0,48;<br>+47,22; | +10,40<br>-7,93 |
| 1.11 | in a) 1,1,2,3-Tetramethyl-1H-benz[e]indolinium-bromid 3,51 g<br>in b) 3,4-Dihydroxybenzaldehyd 1,59 g | weinrot | unbehandelte Haare:<br>Nach dem Färben: | +83,30;<br>+32,40; | -0,48;<br>+37,51; | +10,40<br>+1,86 |
| 1.12 | in a) 1,1,2,3-Tetramethyl-1H-benz[e]indolinium-jodid 4,05 g<br>in b) 3,5-Dimethoxy-4-hydroxy-benzaldehyd 2,10 g | blau-violett | unbehandelte Haare:<br>Nach dem Färben: | +83,30;<br>+36,10; | -0,48;<br>+32,89; | +10,40<br>-17,69 |

Tabelle 2: (Fortsetzung)

| Nr. | a) Enaminhaltige Komponente A1<br>b) Aldehydhaltige Komponente A2 | Farbton nach dem Färben | Farbmeßwerte | L | a | b |
|---|---|---|---|---|---|---|
| 1.13 | in (a) 1,1,2,3-Tetramethyl-1H-benz[e]indolinium-chlorid 2,99 g<br>in (b) 3,4,5-Trihydroxybenzaldehyd Monohydrat 1,98 g | petrol | unbehandelte Haare:<br>Nach dem Färben: | +83,30;<br>+21,51; | -0,48;<br>+11,82; | +10,40<br>-6,08 |
| 1.14 | in a) 1,1,2,3-Tetramethyl-1H-benz[e]indolinium chlorid 3,00 g<br>in b) 3,4-Dihydroxy-benzaldehyd 0,80 g;<br>3-Hydroxy-4-methoxy-benzaldehyd 0,88 g;<br>1-(2-Hydroxyethyl)amino-2-nitro-4-[di(2-hydroxyethyl)amino]-benzol (HC Blue No. 2) 2,00 g | violett | unbehandelte Haare:<br>Nach dem Färben: | +83,30;<br>+21,43; | -0,48;<br>+11,30; | +10,40<br>-6,36 |

Tabelle 2: (Fortsetzung)

| Nr. | a) Enaminhaltige Komponente A1<br>b) Aldehydhaltige Komponente A2 | Farbton nach dem Färben | | Farbmeßwerte L | a | b |
|---|---|---|---|---|---|---|
| 1.15 | (a) 1,2,3,3-Tetramethyl-3H-indoliniumchlorid 2,42 g | intensiv-rot | unbehandelte Haare: | +83,30; | -0,48; | +10,40 |
| | (b) 4-Hydroxy-3-methoxy-benzaldehyd 1,76 g | | Nach dem Färben: | +25,32; | +58,19; | +12,67 |
| 1.16 | a) 1,2,3,3-Tetramethyl-3H-indoliniumchlorid 2,42 g | intensiv-orange | unbehandelte Haare: | +83,30; | -0,48; | +10,40 |
| | b) 4-Hydroxybenzaldehyd 1,43 g | | Nach dem Färben: | +44,34; | +73,22; | +42,70 |
| 1.17 | a) 1,2,3,3-Tetramethyl-3H-indoliniumchlorid 2,42 g | intensiv-gelb | unbehandelte Haare: | +83,30; | -0,48; | +10,40 |
| | b) 3-Hydroxy-4-methoxy-benzaldehyd 1,76 g | | Nach dem Färben: | +58,96; | +33,01; | +66,62 |

Tabelle 2: (Fortsetzung)

| Nr. | a) Enaminhaltige Komponente A1<br>b) Aldehydhaltige Komponente A2 | Farbton nach dem Färben | | Farbmeßwerte L | a | b |
|---|---|---|---|---|---|---|
| 1.18 | a) 1,2,3,3-Tetramethyl-3H-indoliniumchlorid 2,42 g | intensiv-rosa | unbehandelte Haare: | +83,30; | -0,48; | +10,40 |
| | b) 4-Dimethylaminobenzaldehyd 1,72 g | | Nach dem Färben: | +45,75; | +77,26; | +0,54 |
| 1.19 | a) 1,2,3,3-Tetramethyl-3H-indoliniumchlorid 2,42 g | intensiv-weinrot | unbehandelte Haare: | +83,30; | -0,48; | +10,40 |
| | b) 3,4-Dihydroxybenzaldehyd 1,59 g | | Nach dem Färben: | +21,81; | +37,19; | +5,12 |
| 1.20 | a) 1,2,3,3-Tetramethyl-3H-indoliniumchlorid 2,42 g | intensiv-violett | unbehandelte Haare: | +83,30; | -0,48; | +10,40 |
| | b) 3,5-Dimethoxy-4-hydroxy-benzaldehyd 2,10 g | | Nach dem Färben: | +24,02; | +49,64; | -8,16 |

Tabelle 2: (Fortsetzung)

| Nr. | a) Enaminhaltige Komponente A1<br>b) Aldehydhaltige Komponente A2 | Farbton nach dem Färben | | Farbmeßwerte L | a | b |
|---|---|---|---|---|---|---|
| 1.21 | a) 1,2,3,3-Tetramethyl-3H-indolinium bromid 2,93 g<br>b) 3,4-Dihydroxy-benzaldehyd 0,80 g,<br>3-Hydroxy-4-methoxy-benzaldehyd 0,88 g,<br>1-(2-Hydroxyethyl)amino-2-nitro-4-[di(2-hydroxyethyl)amino]-benzol (HC Blue No. 2) 2,00 g | intensiv-rotbraun | unbehandelte Haare:<br>Nach dem Färben: | +83,30;<br>+19,86; | -0,48;<br>+16,36; | +10,40<br>+3,51 |

**Beispiele 2.1 bis 2.20: Haarfärbemittel**

| **Enaminhaltige Komponente A1** | |
|---|---|
| Enamin der Formel (I) | Mengenangaben gemäß Tabelle 1 |
| 6-O-Palmitoyl-L-ascorbinsäure | 0,3 g |
| Cetylstearylalkohol | 12,0 g |
| Laurylethersulfat, 28%ige wäßrige Lösung | 10,0 g |
| Ethanol | 23,0 g |
| Wasser, vollentsalzt | ad 100,0 g |

Der Cetylstearylalkohol wird bei 80 °C geschmolzen. Das Laurylethersulfat wird mit 95 % des Wassers auf 80 °C erhitzt und zum geschmolzenen Cetylstearylalkohol gegeben und gerührt bis sich eine Creme ergibt. Bei Raumtemperatur wird Verbindung (I), mit dem Ethanol und dem restlichen Wasser sowie der 6-O-Palmitoyl-L-ascorbinsäure versetzt, zugegeben. Der pH-Wert der Creme wird mit 10%iger Natronlauge auf 11,0 eingestellt.

| **Aldehydhaltige Komponente A2** | |
|---|---|
| Aldehydverbindung | Mengenangaben gemäß Tabelle 1 |
| Cetylstearylalkohol | 12,0 g |
| Laurylethersulfat, 28%ige wäßrige Lösung | 10,0 g |
| 6-O-Palmitoyl-L-ascorbinsäure | 0,3 g |
| Ethanol | 23,0 g |
| Wasser, vollentsalzt | ad 100,0 g |

Der Cetylstearylalkohol wird bei 80 °C geschmolzen. Das Laurylethersulfat wird mit 95 % des Wassers auf 80 °C erhitzt und zum geschmolzenen Cetylstearylalkohol gegeben und gerührt bis sich eine Creme ergibt. Bei Raumtemperatur wird der Aldehyd, mit dem Ethanol und dem restlichen Wasser sowie der 6-O-Palmitoyl-L-ascorbinsäure versetzt, zugegeben. Der pH-Wert der Creme wird mit 10 %iger wässriger Milchsäure auf 4,0 eingestellt.

Die Komponente A1 und die Komponente A2 werden im Verhältnis 1:1 miteinander vermischt. Das so erhaltene gebrauchsfertige Haarfärbemittel wird auf das Haar aufgetragen und mit einem Pinsel gleichmäßig verteilt. Nach einer Einwirkungszeit von 30 Minuten bei 40 °C wird das Haar mit einem Shampoo gewaschen, anschließend mit lauwarmem Wasser gespült und sodann getrocknet.

Das Haar kann zu einem beliebigen Zeitpunkt (beispielsweise nach mehreren Tagen oder Wochen) mit einer 5 %igen Natriumsulfit-Lösung mit einem pH-Wert von 5 (Komponente B) innerhalb von 20 Minuten bei 40 °C wieder vollständig entfärbt werden.

Die Färbe- und Entfärbeergebnisse sind in der nachfolgenden Tabelle 3 zusammengefaßt.

Tabelle 3:

| Nr. | in A1 enthaltenes Enamin (I); in A2 enthaltener Aldehyd | Farbton nach der Färbung | Farbmeßwerte L a b | Entfärbe -grad(%) |
|---|---|---|---|---|
| 2.1 | in (A1) 1,2,3,3-Tetramethyl-3H-indolinium chlorid 2,42 g; in (A2) 4-Hydroxy-3-methoxy-benzaldehyd 1,76 g | intensiv rot | unbehandelte Haare: +83,30; -0,48; +10,40<br>Nach dem Färben: +34,04; +68,92; +19,11<br>Nach dem Entfärben: +84,19; +9,28; +18,85 | 86 |
| 2.2 | in (A1) 1,2,3,3-Tetramethyl-3H-indolinium chlorid 2,42 g; in (A2) 4-Hydroxybenzaldehyd 1,43 g | intensiv orange | unbehandelte Haare: +83,30; -0,48; +10,40<br>Nach dem Färben: +52,07; +69,44; +56,39<br>Nach dem Entfärben: +82,72; +11,60; +17,04 | 86 |
| 2.3 | in (A1) 1,2,3,3-Tetramethyl-3Hindolinium chlorid 2,42 g; in (A2) 3-Hydroxy-4-methoxy-benzaldehyd 1,76 g | intensiv gelb | unbehandelte Haare: +83,30; -0,48; +10,40<br>Nach dem Färben: +67,01; +26,58; +78,16<br>Nach dem Entfärben: +84,10; +10,52; +17,29 | 84 |

Tabelle 3: (Fortsetzung)

| Nr. | in A1 enthaltenes Enamin (I); in A2 enthaltener Aldehyd | Farbton nach der Färbung | | Farbmeßwerte L | a | b | Entfärbe -grad(%) |
|---|---|---|---|---|---|---|---|
| 2.4 | in (A1) 1,2,3,3-Tetramethyl-3H-indolinium chlorid 2,42 g; in (A2) 3,5-Dimethoxy-4-hydroxy-benzaldehyd 2,10 g | intensiv violett | unbehandelte Haare: | +83,30; | -0,48; | +10,40 | |
| | | | Nach dem Färben: | +26,06; | +52,34; | -6,83 | |
| | | | Nach dem Entfärben: | +80,48; | +7,83; | +15,56 | 88 |
| 2.5 | in (A1) 2-Methylen-1,3,3-trimethyl-indolin 2,00 g; in (A2) 3,4-Dihydroxybenzaldehyd 1,59 g | intensiv weinrot | unbehandelte Haare: | +83,30; | -0,48; | +10,40 | |
| | | | Nach dem Färben: | +24,17; | +41,98; | +8,34 | |
| | | | Nach dem Entfärben: | +80,06; | +9,09; | +19,41 | 82 |
| 2.6 | in (A1) 1,1,2,3-Tetramethyl-1H-benz[e]indolinium iodid 4,05 g; in (A2) 3,5-Dimethoxy-4-hydroxy-benzaldehyd 1,59 g | schwarz | unbehandelte Haare: | +34,41; | +7,27; | +13,78 | |
| | | | Nach dem Färben: | +23,05; | +7,19; | +0,35 | |
| | | | Nach dem Entfärben: | +30,81; | +8,26; | +12,45 | 85 |

Tabelle 3: (Fortsetzung)

| Nr. | in A1 enthaltenes Enamin (I); in A2 enthaltener Aldehyd | Farbton nach der Färbung | | Farbmeßwerte L | a | b | Entfärbe -grad(%) |
|---|---|---|---|---|---|---|---|
| 2.7 | in (A1) 1,1,2,3-Tetramethyl-1H-benz[e]indolinium-chlorid 2,99 g; in (A2) 4-Hydroxy-3-methoxy-benzaldehyd 1,76 g | | unbehandelte Haare: | +83,30; | -0,48; | +10,40 | |
| | | rot | Nach dem Färben: | +47,43; | +50,09; | +3,75 | |
| | | nach der Entfärbung: | weiss | | | | |
| 2.8 | in (A1) 1,1,2,3-Tetramethyl-1H-benz[e]indolinium-chlorid 2,99 g; in (A2) 4-Hydroxybenzaldehyd 1,43 g | | unbehandelte Haare: | +83,30; | -0,48; | +10,40 | |
| | | orange | Nach dem Färben: | +60,27; | +53,71; | +43,96 | |
| | | nach der Entfärbung: | weiss | | | | |

Tabelle 3: (Fortsetzung)

| Nr. | in A1 enthaltenes Enamin; in A2 enthaltener Aldehyd | Farbton nach der Färbung/ Entfärbung | | Farbmeßwerte L | a | b |
|---|---|---|---|---|---|---|
| 2.9 | in (A1) 1,1,2,3-Tetramethyl-1H-benz[e]indolinium iodid 4,05 g; in (A2) 3-Hydroxy-4-methoxy-benzaldehyd 1,76 g | | unbehandelte Haare: | +83,30; | -0,48; | +10,40 |
| | | gelb | Nach dem Färben: | +68,20; | +22,52; | +57,04 |
| | | nach der Entfärbung: | weiss | | | |
| 2.10 | in (A1) 1,1,2,3-Tetramethyl-1H-benz[e]indolinium-bromid 3,51 g; in (A2) 4-Dimethylaminobenzaldehyd 1,72 g | | unbehandelte Haare: | +83,30; | -0,48; | +10,40 |
| | | rosa | Nach dem Färben: | +52,30; | +47,22; | -7,93 |
| | | nach der Entfärbung: | weiss | | | |

**Tabelle 3:** (Fortsetzung)

| **Nr.** | **in A1 enthaltenes Enamin; in A2 enthaltener Aldehyd** | **Farbton nach der Färbung/ Entfärbung** | | **Farbmeßwerte L** | **a** | **b** |
|---|---|---|---|---|---|---|
| **2.11** | in (A1) 1,1,2,3-Tetramethyl-1H-benz[e]indolinium-chlorid 2,99 g; in (A2) 3,4-Dihydroxybenzaldehyd 1,59 g | | unbehandelte Haare: | +83,30; | -0,48; | +10,40 |
| | | weinrot | Nach dem Färben: | +32,40; | +37,51; | +1,86 |
| | | nach der Entfärbung: | weiss | | | |
| **2.12** | in (A1) 1,1,2,3-Tetramethyl-1H-benz[e]indolinium-chlorid 2,99 g; in (A2) 3,5-Dimethoxy-4-hydroxybenzaldehyd 2,10 g | | unbehandelte Haare: | +83,30; | -0,48; | +10,40 |
| | | blau-violett | Nach dem Färben: | +36,10; | +32,89; | -17,69 |
| | | nach der Entfärbung: | weiss | | | |

Tabelle 3: (Fortsetzung)

| Nr. | in A1 enthaltenes Enamin; in A2 enthaltener Aldehyd | Farbton nach der Färbung/ Entfärbung | | Farbmeßwerte L | a | b |
|---|---|---|---|---|---|---|
| 2.13 | in (A1) 1,2,3,3-Tetramethyl-3H-indolinium chlorid 2,42 g in (A2) 4-Methylimidazol-5-carboxaldehyd 1,27 g | intensiv gelb | unbehandelte Haare: | +83,30; | -0,48; | +10,40 |
| | | | Nach dem Färben: | +81,27; | -4,04; | +94,57 |
| | | nach der Entfärbung: | weiss | | | |
| 2.14 | in (A1) 1,2,3,3-Tetramethyl-3H-indolinium chlorid 2,42 g in (A2) 4-Hydroxy-2-methoxybenzaldehyd 1,75 g | intensiv orange | unbehandelte Haare: | +83,30; | -0,48; | +10,40 |
| | | | Nach dem Färben: | +47,00; | +71,71; | +47,92 |
| | | nach der Entfärbung: | weiss | | | |

Tabelle 3: (Fortsetzung)

| Nr. | in A1 enthaltenes Enamin; in A2 enthaltener Aldehyd | Farbton nach der Färbung/ Entfärbung | | Farbmeßwerte L a b |
|---|---|---|---|---|
| 2.15 | in (A1) 1,2,3,3-Tetramethyl-3H-indolinium chlorid 2,42 g; in (A2) 3,5-Dimethyl-4-hydroxybenzaldehyd 1,73 g | intensiv rot | unbehandelte Haare: | +83,30; -0,48; +10,40 |
| | | | Nach dem Färben: | +39,51; +72,92; +24,40 |
| | | nach der Entfärbung: | weiss | |
| 2.16 | in (A1) 1,2,3,3-Tetramethyl-3H-indolinium chlorid 2,42 g; in (A2) 4-Dimethylamino-2-methoxy-benzaldehyd 2,10 | intensiv rosa | unbehandelte Haare: | +83,30; -0,48; +10,40 |
| | | | Nach dem Färben: | +37,36; +76,29; +6,25 |
| | | nach der Entfärbung: | schwach rosa | |

Tabelle 3: (Fortsetzung)

| Nr. | in A1 enthaltenes Enamin; in A2 enthaltener Aldehyd | Farbton nach der Färbung/ Entfärbung | | Farbmeßwerte L | a | b |
|---|---|---|---|---|---|---|
| 2.17 | in (A1) 1,2,3,3-Tetramethyl-3H-indolinium chlorid 2,42 g; in (A2) 3,4,5-Trihydroxybenzaldehyd 2,0 g | intensiv rot-violett | unbehandelte Haare: | +83,30; | -0,48; | +10,40 |
| | | | Nach dem Färben: | +19,53; | +21,90; | -2,69 |
| | | nach der Entfärbung: | weiss | | | |
| 2.18 | in (A1) 1,1,2,3-Tetramethyl-1H-benz[e]indolinium-chlorid 2,99 g; in (A2) 3,4,5-Trihydroxybenzaldehyd 2,0 g | blau | unbehandelte Haare: | +83,30; | -0,48; | +10,40 |
| | | | Nach dem Färben: | +21,51; | +11,82; | -6,08 |
| | | nach der Entfärbung: | weiss | | | |

Tabelle 3: (Fortsetzung)

| Nr. | in A1 enthaltenes Enamin; in A2 enthaltener Aldehyd | Farbton nach der Färbung/ Entfärbung | | Farbmeßwerte L | a | b |
|---|---|---|---|---|---|---|
| 2.19 | in (A1) 5-Chloro-2-methylen-1,3,3-trimethylindolin 3,36 g; in (A2) 3,4-Dihydroxybenzaldehyd 1,59 g | | unbehandelte Haare: | +83,30; | -0,48; | +10,40 |
| | | weinrot | Nach dem Färben: | +26,67; | +45,74; | -4,84 |
| | | nach der Entfärbung: | weiss | | | |
| 2.20 | in (A1) 5-Chloro-2-methylen-1,3,3-trimethylindolin 3,36 g; in (A2) 4-Hydroxybenzaldehyd 1,43 g | | unbehandelte Haare: | +83,30; | -0,48; | +10,40 |
| | | orange | Nach dem Färben: | +54,25; | +67,96; | +39,02 |
| | | nach der Entfärbung: | weiss | | | |

**Beispiele 3.1 bis 3.2: Haarfärbemittel**

| **Enaminhaltige Komponente A1** | |
|---|---|
| 1,3,3-Trimethyl-2-methylen-indolin | 2,0 g |
| Cetylstearylalkohol | 12,0 g |
| Stearylalkohol-polyethylenglykolether mit 20 Mol Ethylenoxid im Molekül (Steareth-20) | 1,4 g |
| Isopropanol | 20,0 g |
| Wasser, vollentsalzt | ad 100,0 g |
| **Aldehydhaltige Komponente A2 (3.1)** | |
| 4-Hydroxy-3-methoxybenzaldehyd | 1,76 g |
| Cetylstearylalkohol | 12,00 g |
| Stearylalkohol-polyethylenglykolether mit 20 Mol Ethylenoxid im Molekül (Steareth-20) | 1,40 g |
| Isopropanol | 20,00 g |
| Wasser, vollentsalzt | ad 100,00 g |
| **Aldehydhaltige Komponente A2 (3.2)** | |
| 4-Hydroxybenzaldehyd | 1,41 g |
| Cetylstearylalkohol | 12,00 g |
| Stearylalkohol-polyethylenglykolether mit 20 Mol Ethylenoxid im Molekül (Steareth-20) | 1,40 g |
| Isopropanol | 20,00 g |
| Wasser, vollentsalzt | ad 100,00 g |

5 g der enaminhaltigen Komponente A1 werden mit 5 g der aldehydhaltigen Komponente (A2/3.1) bzw. (A2/3.2) vermischt. Das erhaltene gebrauchsfertige Haarfärbemittel wird auf gebleichte hellbraune Haare aufgetragen und mit einem Pinsel gleichmäßig verteilt. Nach einer Einwirkungszeit von 30 Minuten bei 40 °C wird das Haar mit lauwarmem Wasser gespült und sodann getrocknet.

**Beispiele 3.3 bis 3.4: Haarfärbemittel**

| **Enaminhaltige Komponente A1** | |
|---|---|
| 1,2,3,3-Tetramethyl-3H-indoliniumchlorid | 2,42 g |
| 6-O-Palmitoyl-L-ascorbinsäure | 0,30 g |
| Cetylstearylalkohol | 12,00 g |
| Laurylethersulfat 28 %ig | 10,00 g |
| Ethanol | 23,0 g |
| Wasser, vollentsalzt | ad 100,0 g |

Der Cetylstearylalkohol wird bei 80 °C geschmolzen. Das Laurylethersulfat wird mit 95 % des Wassers auf 80 °C erhitzt und zum geschmolzenen Cetylstearylalkohol gegeben und gerührt bis sich eine Creme ergibt. Bei Raumtemperatur wird das 1,2,3,3-Tetramethyl-3H-indoliniumchlorid mit dem restlichen Wasser und dem Alkohol und mit der 6-O-Palmitoyl-L-ascorbinsäure versetzt, zugegeben. Der pH-Wert der Creme wird mit 10 %iger wässriger NaOH auf 12,0 eingestellt.

| **Aldehydhaltige Komponente A2 (3.3)** | |
|---|---|
| 4-Hydroxy-3-methoxybenzaldehyd | 1,76 g |
| Cetylstearylalkohol | 12,00 g |
| Laurylethersulfat 28 %ig | 10,00 g |
| Ethanol | 23,0 g |

(fortgesetzt)

| **Aldehydhaltige Komponente A2 (3.3)** | |
|---|---|
| Wasser, vollentsalzt | ad 100,0 g |
| **Aldehydhaltige Komponente A2 (3.4)** | |
| 4-Hydroxy-benzaldehyd | 1,416 g |
| Cetylstearylalkohol | 12,00 g |
| Laurylethersulfat 28 %ig | 10,00 g |
| Ethanol | 23,0 g |
| Wasser, vollentsalzt | ad 100,0 g |

Der Cetylstearylalkohol wird bei 80 °C geschmolzen. Das Laurylethersulfat wird mit 95 % des Wassers auf 80 °C erhitzt und zum geschmolzenen Cetylstearylalkohol gegeben und gerührt bis sich eine Creme ergibt. Bei Raumtemperatur wird der Aldehyd, mit dem restlichen Wasser und dem Alkohol versetzt, zugegeben. Der pH-Wert der Creme (A2/3.3) bzw. (A2/3.4) wird mit 10 %iger wässriger Milchsäure auf 4,0 eingestellt.

5 g der das 1,2,3,3-Tetramethyl-3H-indoliniumchlorid enthaltenden Komponente A1 werden mit 5 g der aldehydhaltigen Komponente (A2/3.3) bzw. (A2/3.4) vermischt. Das erhaltene gebrauchsfertige Haarfärbemittel wird auf gebleichte hellbraune Haare aufgetragen und mit einem Pinsel gleichmäßig verteilt. Nach einer Einwirkungszeit von 30 Minuten bei 40 °C wird das Haar mit lauwarmem Wasser gespült und sodann getrocknet.

Beim sich anschließenden Waschtest werden die gefärbten Strähnen jeweils 5mal mit einem Shampoo gewaschen, mit Wasser gespült und getrocknet. Nach jedem Waschvorgang werden die L*a*b*-Werte bestimmt. Die Ergebnisse sind in Tabelle 4 zusammengefaßt.

**Tabelle 4:** Färbe-Resultate und Waschstabilität

| Nr. | Enaminhaltige Komponente A1 / Aldehydhaltige Komponente A2 | Farbton nach dem Färben | Farbmeßwerte | L | a | b |
|---|---|---|---|---|---|---|
| 3.1 | in A1: 1,3,3-Trimethyl-2-methylen-indolin 2,0 g<br>in A2: 4-Hydroxy-3-methoxy-benzaldehyd 1,76 g | intensiv-rot | Vor dem Färben: | 60,31; | +11,53; | +31,63 |
| | | | Nach dem Färben: | 27,60; | +49,86; | +7,56 |
| | | | Nach 1x Waschen: | 28,44; | +49,48; | +6,63 |
| | | | Nach 2x Waschen: | 28,24; | +49,14; | +5,55 |
| | | | Nach 3x Waschen: | 28,66; | +49,78; | +5,47 |
| | | | Nach 4x Waschen: | 28,27; | +49,25; | +6,39 |
| | | | Nach 5x Waschen: | 28,16; | +49,15; | +6,34 |

Tabelle 4: (Fortsetzung)

| Nr. | Enaminhaltige Komponente A1<br>Aldehydhaltige Komponente A2 | Farbton nach dem Färben | Farbmeßwerte | L | a | b |
|---|---|---|---|---|---|---|
| 3.2 | in A1: 1,3,3-Trimethyl-2-methylen-indolin 2,0 g<br>in A2: 4-Hydroxybenzaldehyd 1,41 g | intensiv-orange | Vor dem Färben: | 60,31; | +11,53; | +31,63 |
| | | | Nach dem Färben: | 40,74; | +55,06; | +34,51 |
| | | | Nach 1x Waschen: | 39,86; | +54,07; | +31,17 |
| | | | Nach 2x Waschen: | 39,54; | +53,48; | +29,91 |
| | | | Nach 3x Waschen: | 40,54; | +53,43; | +31,95 |
| | | | Nach 4x Waschen: | 39,39; | +52,29; | +28,72 |
| | | | Nach 5x Waschen: | 39,75; | +51,24; | +29,67 |

Tabelle 4: (Fortsetzung)

| Nr. | Enaminhaltige Komponente A1<br>Aldehydhaltige Komponente A2 | Farbton nach dem Färben | | Farbmeßwerte L | a | b |
|---|---|---|---|---|---|---|
| 3.3 | in A1: 1,2,3,3-Tetramethyl-3H-indolinium chlorid 2,42 g<br>in A2: 4-Hydroxy-3-methoxy-benzaldehyd 1,76 g | intensiv-rot | Vor dem Färben: | 60,31; | +11,53; | +31,63 |
| | | | Nach dem Färben: | 27,60; | +49,86; | +7,56 |
| | | | Nach 1x Waschen: | 28,44; | +49,48; | +6,63 |
| | | | Nach 2x Waschen: | 28,24; | +49,14; | +5,55 |
| | | | Nach 3x Waschen: | 28,66; | +49,78; | +5,47 |
| | | | Nach 4x Waschen: | 28,27; | +49,25; | +6,39 |
| | | | Nach 5x Waschen: | 28,16; | +49,15; | +6,34 |

Tabelle 4: (Fortsetzung)

| Nr. | Enaminhaltige Komponente A1 Aldehydhaltige Komponente A2 | Farbton nach dem Färben | | Farbmeßwerte L | a | b |
|---|---|---|---|---|---|---|
| 3.4 | in A1: 1,2,3,3-Tetramethyl-3H-indolinium chlorid 2,42 g in A2: 4-Hydroxybenzaldehyd 1,41 g | intensiv-orange | Vor dem Färben: | 60,31; | +11,53; | +31,63 |
| | | | Nach dem Färben: | 40,74; | +55,06; | +34,51 |
| | | | Nach 1x Waschen: | 39,86; | +54,07; | +31,17 |
| | | | Nach 2x Waschen: | 39,54; | +53,48; | +29,91 |
| | | | Nach 3x Waschen: | 40,54; | +53,43; | +31,95 |
| | | | Nach 4x Waschen: | 39,39; | +52,29; | +28,72 |
| | | | Nach 5x Waschen: | 39,75; | +51,24; | +29,67 |

Die in den vorliegenden Beispielen angegebenen L*a*b*-Farbmesswerte wurden mit einem Farbmessgerät der Firma Minolta, Typ Chromameter II, ermittelt.
Hierbei steht der L-Wert für die Helligkeit (das heißt je geringer der L-Wert ist, umso größer ist die Farbintensität), während der a-Wert ein Maß für den Rotanteil ist (das heißt je größer der a-Wert ist, umso größer ist der Rotanteil). Der b-Wert ist ein Maß für den Blauanteil der Farbe, wobei der Blauanteil umso größer ist, je negativer der b-Wert ist.

Der Wert D gibt die Farbdifferenz an, die zwischen den unbehandelten und den gefärbten bzw. entfärbten Strähnchen besteht. Er wird folgendermaßen bestimmt:

$$D = \sqrt{(L_i-L_0)^2 + (a_i-a_0)^2 + (b_i-b_0)^2}$$

wobei $L_0$, $a_0$ und $b_0$ die Farbmesswerte von unbehandeltem Haar und $L_i$, $a_i$ und $b_i$ die Werte des behandelten Haares darstellen. Die Entfärberate in Prozent wurde folgendermaßen ermittelt:

$$\text{Entfärbe-\%} = [1 - (\text{D nach Entfärbung/D nach Färbung})] \times 100.$$

Alle Prozentangaben in der vorliegenden Anmeldung stellen, sofern nicht anders angegeben, Gewichtsprozente dar.

## Patentansprüche

1. Mittel zur Färbung von Fasern, welches durch Vermischen zweier Komponenten erhalten wird, **dadurch gekennzeichnet, daß** die eine Komponente (A1) mindestens ein Enamin der Formel (I) oder dessen physiologisch verträgliches Salz,

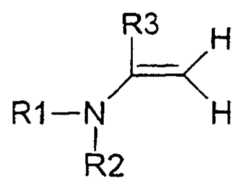
(I)

in der **R1** gleich einem ein- oder mehrkernigen aromatischen Rest, insbesondere einem gegebenenfalls mit einer C1- bis C4-Alkylgruppe, C1- bis C4-Hydroxyalkylgruppe, Hydroxygruppe, Methoxygruppe, Dialkylaminogruppe oder Halogengruppe substituierten 5- gliedrigen oder 6-gliedrigen Arylrest, vorzugsweise einem Phenylrest, oder einem 5-gliedrigen oder 6-gliedrigen Heterozyklus, vorzugsweise einem Pyridylrest, oder einem Naphthylrest ist; **R2** gleich einer geradkettigen oder verzweigten C1- bis C8-Alkylgruppe, einer geradkettigen oder verzweigten C1- bis C8-Hydroxyalkylgruppe oder einer C1- bis C8-Alkoxyalkylgruppe, wobei zwischen den C-Atomen der Alkylkette Sauerstoffatome sitzen können, ist und R3 gleich einer geradkettigen oder verzweigten C1- bis C8-Alkylgruppe, einer C1- bis C8-Alkoxyalkylgruppe, einer geradkettigen oder verzweigten C1- bis C8-Alkylengruppe, einer C1- bis C8-Alkoxyalkylengruppe, -O-, -NH-, -$NR_4$- oder -S- ist, mit $\mathbf{R_4}$ gleich einer Alkylgruppe, Alkoxyalkylgruppe, Hydroxyalkylgruppe oder Wasserstoff, wobei die Reste **R1** und **R3** gemeinsam mit dem Stickstoffatom und dem Kohlenstoffatom der Enamingrundstruktur eine zyklische Verbindung bilden können, enthält und die andere Komponente (A2) mindestens eine Carbonylverbindung enthält.

2. Mittel nach Anspruch 1, **dadurch gekennzeichnet, daß** das Enamin der Formel (I) oder dessen Salz ausgewählt ist aus Verbindungen der Formeln (II) bis (IX),

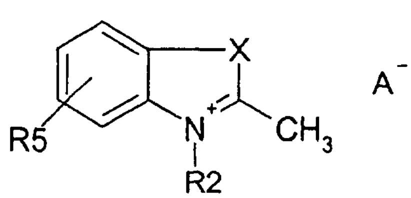
(II)

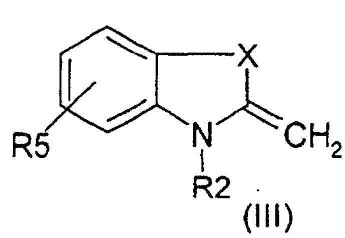
(III)

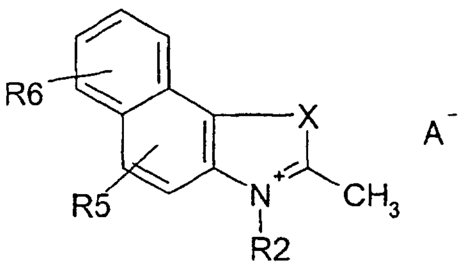

(IV)

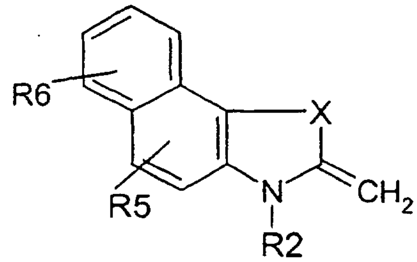

(V)

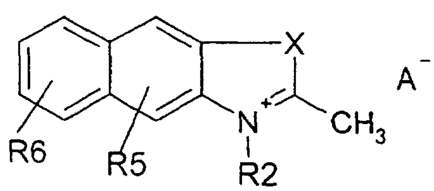

(VI)

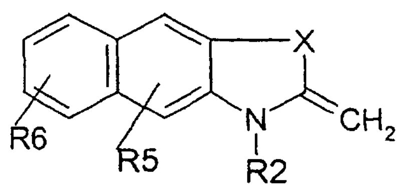

(VII)

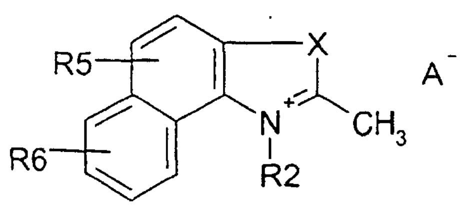

(VIII)

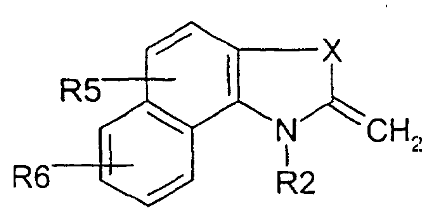

(IX)

in denen **X** gleich einem mit zwei C1- bis C4-Alkylgruppen oder einer C1-bis C4-Alkylgruppe und einer Hydroxygruppe substituierten C-Atom, einem Schwefelatom, einem alkylierten oder nichtalkylierten Stickstoffatom, oder einem Sauerstoffatom ist; und **R2** gleich einer geradkettigen oder verzweigten C1- bis C8-Alkylgruppe, einer geradkettigen oder verzweigten C1- bis C8-Hydroxyalkylgruppe oder einer C1- bis C8-Alkoxyalkylgruppe ist, wobei zwischen den C-Atomen der Alkylkette Sauerstoffatome sitzen können; und **R5** und **R6** unabhängig voneinander gleich Wasserstoff, einer geradkettigen oder verzweigten C1-bis C4-Alkylgruppe, einer geradkettigen oder verzweigten C1- bis C4-Hydroxyalkylgruppe, einer Hydroxygruppe, einer Methoxygruppe, einer Aminogruppe, einer Dialkylaminogruppe oder einem Halogenatom sind; und **A$^-$** gleich Chlorid, Bromid, Iodid, Hydrogensulfat, Monomethylsulfat, Sulfat, Hexafluor-phosphat, Hexafluorantimonat, Tetrafluorborat, Tetraphenylborat ist.

**3.** Mittel nach Anspruch 2, **dadurch gekennzeichnet, daß** das Enamin der Formeln (II) bis (IX) ausgewählt ist aus 3-Ethyl-2-methylenbenzothiazolin, 2-Methylen-1,3,3-trimethyl-indolin, 1,2,3,3-Tetramethyl-3Hindolinium-chlorid, 1,2,3,3-Tetramethyl-3H-indolinium-bromid, 1,2,3,3-Tetramethyl-3H-indolinium-iodid, 1,2,3,3-Tetramethyl-3H-indolinium-sulfat, 1,2,3,3-Tetramethyl-3H-indolinium-hydrogensulfat, 1,2,3,3-Tetramethyl-3H-indolinium-methylsulfat, 1,2,3,3-Tetramethyl-3H-indoliniumhexafluorphosphat, 1,2,3,3-Tetramethyl-3H-indolinium hexafluorantimonat,

1,2,3,3-Tetramethyl-3H-indolinium-tetrafluorborat, 5-Chloro-2-methylen-1,3,3-trimethyl-indolin oder dessen Salze, 1-(2-Hydroxyethyl)-3,3-dimethyl-2-methylen-indolin oder dessen Salze, 1,1,2,3-Tetramethyl-1H-benz[e]indolinium-chlorid, 1,1,2,3-Tetramethyl-1Hbenz[e]indolinium-bromid, 1,1,2,3-Tetramethyl-1H-benz[e]indolinium-iodid, 1,1,2,3-Tetramethyl-1H-benz[e]indolinium-sulfat, 1,1,2,3-Tetramethyl-1Hbenz[e]indolinium-hexafluorphosphat, 1,1,2,3-Tetramethyl-1Hbenz[e]indolinium-methylsulfat, 1,1,2,3-Tetramethyl-1H-benz[e]indoliniumhexafluorantimonat und 1,1,2,3-Tetramethyl-1 H-benz[e]indoliniumtetrafluorborat.

**4.** Mittel nach einem der Ansprüche 1 bis 3, **dadurch gekennzeichnet, daß** die Carbonylverbindung ausgewählt ist aus Vanillin (4-Hydroxy-3-methoxybenzaldehyd), Isovanillin (3-Hydroxy-4-methoxy-benzaldehyd), 3,4-Dihydroxybenzaldehyd, 4-Hydroxybenzaldehyd, 3,5-Dimethoxy-4-hydroxy-benzaldehyd, 4-Dimethylaminobenzaldehyd, 4-Methyl-5-imidazolcarboxaldehyd, 4- Dimethylamino-zimtaldehyd, 4-Hydroxy-2-methoxybenzaldehyd, 3,5-Dimethyl-4-hydroxybenzaldehyd, 4-Dimethylamino-2-methoxybenzaldehyd, 2-Hydroxybenzaldehyd, 4-Hydroxy-1-naphthaldehyd, 4-Methoxy-1-naphthaldehyd, 4-Dimethylamino-1-naphthaldehyd, 4'-Hydroxy-biphenyl-1-carbaldehyd, 2-Hydroxy-3-methoxybenzaldehyd, 2,4-Dihydroxybenzaldehyd, 3,4-Dihydroxy-benzaldehyd, 2,5-Dihydroxybenzaldehyd, 2,3,4-Trihydroxybenzaldehyd, 3,4,5-Trihydroxybenzaldehyd, 2,4,6-Trihydroxybenzaldehyd, 2,4-Dimethoxybenzaldehyd, 2,3-Dimethoxybenzaldehyd, 2,5-Dimethoxy-benzaldehyd, 3,5-Dimethoxybenzaldehyd, 3,4-Dimethoxybenzaldehyd, Indol-3-carbaldehyd, Benzol-1,4-dicarbaldehyd, 4-Ethoxybenzaldehyd, 2-Methyl-1,4-naphthochinon, 4-Carboxybenzaldehyd, 4-Hydroxy-3-methoxyzimtaldehyd, 3,5-Dimethoxy-4-hydroxyzimtaldehyd, 3-Methoxy-4-(1-pyrrolidinyl)-benzaldehyd, 4-Diethylamino-3-methoxybenzaldehyd, 1,2-Phthaldialdehyd, Pyrrol-2-aldehyd, Thiophen-2-aldehyd, Thiophen-3-aldehyd, Chromone-3-carboxaldehyd, 6-Methyl-4-oxo-1(4H)-benzopyran-3-carbaldehyd, N-Methylpyrrol-2-aldehyd, 5-Methylfurfural, 6-Hydroxychromen-3-carboxaldehyd, 6-Methylindol-3-carboxaldehyd, 4-Dibutylaminobenzaldehyd, N-Ethylcarbazol-3-aldehyd, 4-Diethylamino-2-hydroxybenzaldehyd, 3,4-Dimethoxy-5-hydroxybenzaldehyd, 5-(4-(Diethylamino)phenyl)-2,4-pentadienal, 2,3-Thiophendicarboxaldehyd, 2,5-Thiophendicarboxaldehyd, 2-Methoxy-1-naphthaldehyd, 3-Ethoxy-4-hydroxybenzaldehyd, 2-Nitrobenzaldehyd, 3-Nitrobenzaldehyd und 4-Nitrobenzaldehyd.

**5.** Mittel nach einem der Ansprüche 1 bis 4, **dadurch gekennzeichnet, daß** das Enamin und die Carbonylverbindung in dem gebrauchsfertigen Färbemittel jeweils in einer Menge von 0,01 bis 10 Gewichtsprozent enthalten ist.

**6.** Mittel nach einem der Ansprüche 1 bis 5, **dadurch gekennzeichnet, daß** es einen pH-Wert von 3 bis 11 aufweist.

**7.** Mehrkomponenten-Kit zur Färbung und späteren Entfärbung von Fasern, **dadurch gekennzeichnet, daß** er ein Färbemittel A gemäß einem der Ansprüche 1 bis 6 und eine entfärbende Komponente B enthält, wobei die Komponente B mindestens ein Sulfit enthält.

**8.** Mehrkomponenten-Kit nach Anspruch 7, **dadurch gekennzeichnet, daß** das Sulfit ausgewählt ist aus den Ammoniumsulfiten, Alkalisulfiten und Erdalkalisulfiten.

**9.** Mehrkomponenten-Kit nach Anspruch 7 oder 8, **dadurch gekennzeichnet, daß** das Sulfit in der Komponente B in einer Gesamtmenge von 0,1 bis 10 Gewichtsprozent enthalten ist.

**10.** Verfahren zur temporären Färbung von Haaren, bei dem das Haar mit einem Mittel gemäß einem der Ansprüche 1 bis 6 gefärbt wird und zu einem beliebigen späteren Zeitpunkt durch Behandlung der Haare mit einer sulfithaltigen Zubereitung für eine Dauer von 5 bis 60 Minuten bei einer Temperatur von 20 bis 50 °C wieder entfärbt wird.

## Claims

**1.** Agent for colouring fibres, which is obtained by mixing two components, **characterized in that** the one component (A1) comprises at least one enamine of the formula (I) or a physiologically compatible salt thereof,

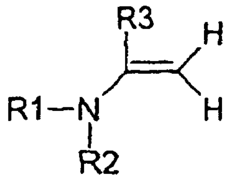

(I)

in which **R1** is a mono- or polynuclear aromatic radical, in particular a 5-membered or 6-membered aryl radical optionally substituted by a C1- to C4-alkyl group, C1-to C4-hydroxyalkyl group, hydroxy group, methoxy group, dialkylamino group or halogen group, preferably a phenyl radical, or a 5-membered or 6-membered heterocycle, preferably a pyridyl radical, or is a naphthyl radical; **R2** is a straight-chain or branched C1- to C8-alkyl group, a straight-chain or branched C1 to C8-hydroxyalkyl group or a C1- to C8-alkoxyalkyl group, where oxygen atoms may sit between the carbon atoms of the alkyl chain, and **R3** is a straight-chain or branched C1- to C8-alkyl group, a C1- to C8-alkoxyalkyl group, a straight-chain or branched C1- to C8-alkylene group, a C1- to C8-alkoxyalkylene group, -O-, -NH-, $-NR_4-$ or -S-, with $\mathbf{R_4}$ being an alkyl group, alkoxyalkyl group, hydroxyalkyl group or hydrogen, where the radicals **R1** and **R3**, together with the nitrogen atom and the carbon atom of the enamine basic structure, can form a cyclic compound, and the other component (A2) comprises at least one carbonyl compound.

2. Agent according to Claim 1, **characterized in that** the enamine of the formula (I) or salts thereof is chosen from compounds of the formulae (II) to (IX),

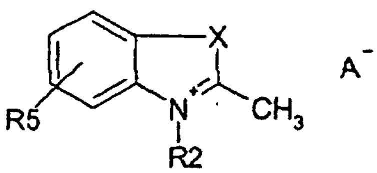

(II)

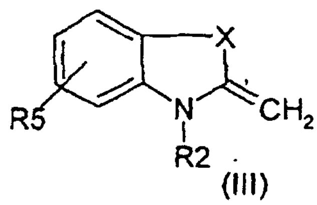

(III)

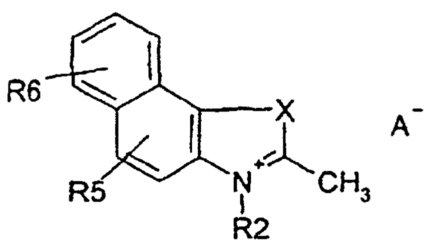

(IV)

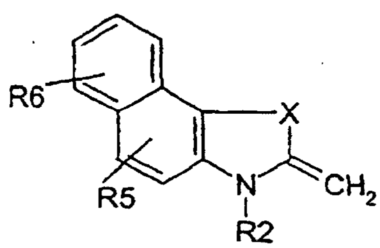

(V)

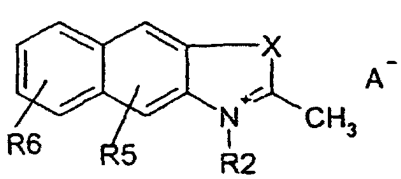

(VI)

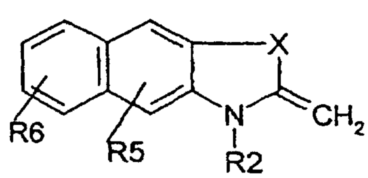

(VII)

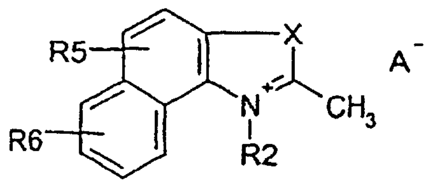

(VIII)

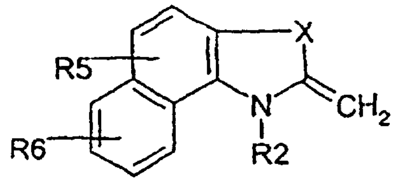

(IX)

in which **X** is a carbon atom substituted by two C1- to C4-alkyl groups or one C1- to C4-alkyl group and one hydroxy group, a sulphur atom, an alkylated or nonalkylated nitrogen atom, or an oxygen atom; and **R2** is a straight-chain or branched C1- to C8-alkyl group, a straight-chain or branched C1- to C8-hydroxyalkyl group or a C1- to C8-alkoxyalkyl group, where oxygen atoms can sit between the carbon atoms of the alkyl chain; and **R5** and **R6**, independently of one another, are hydrogen, a straight-chain or branched C1- to C4-alkyl group, a straight-chain or branched C1- to C4-hydroxyalkyl group, a hydroxy group, a methoxy group, an amino group, a dialkylamino group or a halogen atom; and **A$^-$** is chloride, bromide, iodide, hydrogensulphate, monomethylsulphate, sulphate, hexafluorophosphate, hexafluoroantimonate, tetrafluoroborate, tetraphenylborate.

3. Agent according to Claim 2, **characterized in that** the enamine of the formulae (II) to (IX) is chosen from 3-ethyl-2-methylenebenzothiazoline, 2-methylene-1,3,3-trimethylindoline, 1,2,3,3-tetramethyl-3H-indolinium chloride, 1,2,3,3-tetramethyl-3H-indolinium bromide, 1,2,3,3-tetramethyl-3H-indolinium iodide, 1,2,3,3-tetramethyl-3H-indolinium sulphate, 1,2,3,3-tetramethyl-3H-indolinium hydrogensulphate, 1,2,3,3-tetramethyl-3H-indolinium methylsulphate, 1,2,3,3-tetramethyl-3H-indolinium hexafluorophosphate, 1,2,3,3-tetramethyl-3H-indolinium hexafluoroantimonate, 1,2,3,3-tetramethyl-3H-indolinium tetrafluoroborate, 5-chloro-2-methylene-1,3,3-trimethylindoline or salts thereof, 1-(2-hydroxyethyl)-3,3-dimethyl-2-methylene-indoline or salts thereof, 1,1,2,3-tetramethyl-1H-benz[e]indolinium chloride, 1,1,2,3-tetramethyl-1H-benz[e]indolinium bromide, 1,1,2,3-tetramethyl-1H-benz[e]indolinium iodide, 1,1,2,3-tetramethyl-1H-benz[e]indolinium sulphate, 1,1,2,3-tetramethyl-1H-benz[e]indolinium hexafluorophosphate, 1,1,2,3-tetramethyl-1H-benz[e]indolinium methylsulphate, 1,1,2,3-tetramethyl-1H-benz[e]indolinium hexafluoroantimonate and 1,1,2,3-tetramethyl-1H-benz[e]indolinium tetrafluoroborate.

4. Agent according to one of Claims 1 to 3, **characterized in that** the carbonyl compound is chosen from vanillin (4-hydroxy-3-methoxybenzaldehyde), isovanillin (3-hydroxy-4-methoxybenzaldehyde), 3,4-dihydroxybenzaldehyde, 4-hydroxybenzaldehyde, 3,5-dimethoxy-4-hydroxybenzaldehyde, 4-dimethylaminobenzaldehyde, 4-methyl-5-imidazolcarboxaldehyde, 4-dimethylaminocinnamaldehyde, 4-hydroxy-2-methoxy-benzaldehyde, 3,5-dimethyl-4-hydroxybenzaldehyde, 4-dimethylamino-2-methoxybenzaldehyde, 2-hydroxybenzaldehyde, 4-hydroxy-1-naphthaldehyde, 4-methoxy-1-naphthaldehyde, 4-dimethylamino-1-naphthaldehyde, 4'-hydroxybiphenyl-1-carbaldehyde, 2-hydroxy-3-methoxybenzaldehyde, 2,4-dihydroxybenzaldehyde, 3,4-dihydroxybenzaldehyde, 2,5-dihydroxybenzaldehyde, 2,3,4-trihydroxybenzaldehyde, 3,4,5-trihydroxybenzaldehyde, 2,4,6-trihydroxybenzaldehyde, 2,4-dimethoxybenzaldehyde, 2,3-dimethoxybenzaldehyde, 2,5-dimethoxybenzaldehyde, 3,5-dimethoxybenzaldehyde, 3,4-dimethoxybenzaldehyde, indole-3-carbaldehyde, benzene-1,4-dicarbaldehyde, 4-ethoxybenzaldehyde, 2-methyl-1,4-naphthoquinone, 4-carboxybenzaldehyde, 4-hydroxy-3-methoxycinnamaldehyde, 3,5-dimethoxy-4-hydroxycinnamaldehyde, 3-methoxy-4-(1-pyrrolidinyl)benzaldehyde, 4-diethylamino-3-methoxybenzaldehyde, 1,2-phthaldialdehyde, pyrrole-2-aldehyde, thiophene-2-aldehyde, thiophene-3-aldehyde, chromone-3-carboxaldehyde, 6-methyl-4-oxo-1(4H)benzopyran-3-carbaldehyde, N-methylpyrrole-2-aldehyde, 5-methylfurfural, 6-hydroxychromene-3-carboxaldehyde, 6-methylindole-3-carboxaldehyde, 4-dibutylaminobenzaldehyde, N-ethyl-carbazole-3-aldehyde, 4-diethylamino-2-hydroxybenzaldehyde, 3,4-dimethoxy-5-hydroxybenzaldehyde, 5-(4-(diethylamino)phenyl)-2,4-pentadienal, 2,3-thiophenedicarboxaldehyde, 2,5-thiophenedicarboxaldehyde, 2-methoxy-1-naphthaldehyde, 3-ethoxy-4-hydroxybenzaldehyde, 2-nitrobenzaldehyde, 3-nitrobenzaldehyde and 4-nitrobenzaldehyde.

5. Agent according to one of Claims 1 to 4, **characterized in that** the enamine and the carbonyl compound is present in the ready-to-use colorant in each case in an amount of from 0.01 to 10% by weight.

6. Agent according to one of Claims 1 to 5, **characterized in that** it has a pH of from 3 to 11.

**7.** Multicomponent kit for the colouring and later decolouring of fibres, **characterized in that** it comprises a colorant A according to one of Claims 1 to 6 and a decolouring component B, where the component B comprises at least one sulphite.

**8.** Multicomponent kit according to Claim 7, **characterized in that** the sulphite is chosen from the ammonium sulphites, alkali metal sulphites and alkaline earth metal sulphites.

**9.** Multicomponent kit according to Claim 7 or 8, **characterized in that** the sulphite in component B is present in a total amount of from 0.1 to 10% by weight.

**10.** Method of temporarily colouring hair, in which the hair is coloured with an agent according to one of Claims 1 to 6 and, at any desired later point in time, is decoloured again by treating the hair with a sulphite-containing preparation for a period of from 5 to 60 minutes at a temperature of from 20 to 50°C.

## Revendications

**1.** Agent de teinture de fibres, obtenu par mélange de deux composants, **caractérisé en ce qu'**un composant (A1) contient au moins une énamine de formule (I) ou son sel physiologiquement acceptable

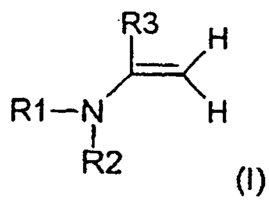

(I)

dans laquelle R1 représente un radical aromatique à un ou plusieurs cycles, en particulier un radical aryle à 5 ou 6 membres, le cas échéant substitué par un groupement alkyle en $C_1$ à $C_4$, hydroxyalkyle en $C_1$ à $C_4$, hydroxy, méthoxy, dialkylamino ou halogène, de préférence un radical phényle, ou un hétérocycle à 5 ou 6 membres, de préférence un radical pyridyle ou un radical naphtyle ; R2 représente un groupement alkyle linéaire ou ramifié en $C_1$ à $C_8$, hydroxyalkyle linéaire ou ramifié en $C_1$ à $C_8$ ou alcoxyalkyle en $C_1$ à $C_8$, des atomes d'oxygène pouvant se trouver entre les atomes de carbone de la chaîne alkyle et R3 représente un groupement alkyle linéaire ou ramifié en $C_1$ à $C_8$, alcoxyalkyle en $C_1$ à $C_8$, alkylène linéaire ou ramifié en $C_1$ à $C_8$, alcoxyalkylène en $C_1$ à $C_8$, -O-, -NH-, -$NR_4$- ou - S- avec $R_4$ représentant un groupement alkyle, alcoxyalkyle, hydroxyalkyle ou hydrogène, les radicaux R1 et R3 pouvant former ensemble un composé cyclique avec l'atome d'azote et l'atome de carbone de la structure de base énamine et l'autre composant (A2) contient au moins un composé carbonyle.

**2.** Agent selon la revendication 1, **caractérisé en ce que** l'énamine de formule (I) ou son sel est choisi parmi les composés des formules (II) à (IX),

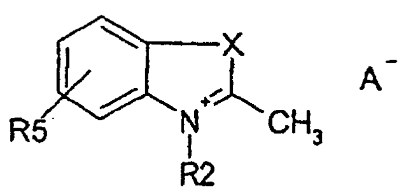

(II)

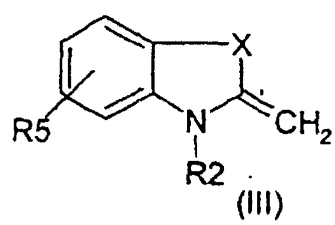

(III)

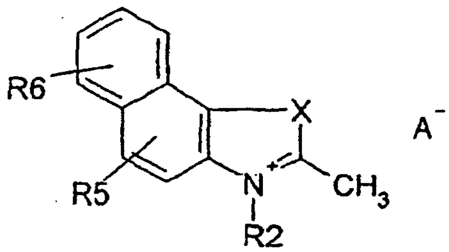

(IV)

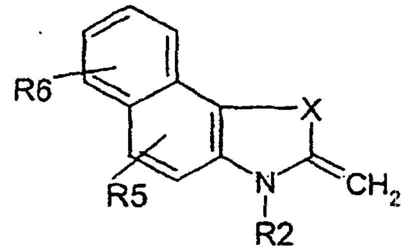

(V)

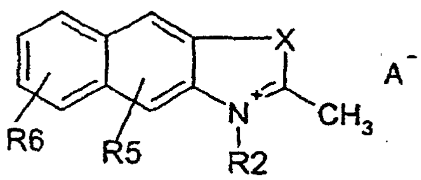

(VI)

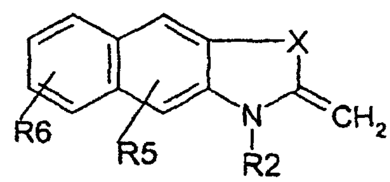

(VII)

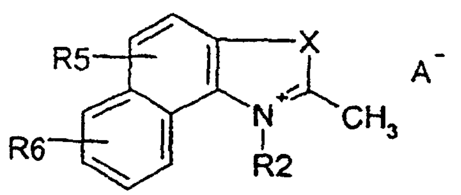

(VIII)

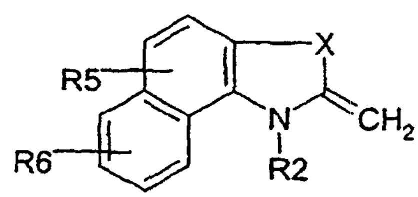

(IX)

dans lesquelles X représente un atome de carbone substitué par deux groupements alkyle en $C_1$ à $C_4$ ou un groupement alkyle en $C_1$ à $C_4$ et un groupement hydroxy, un atome de soufre, un atome d'azote alkylé ou non alkylé ou un atome d'oxygène ; et R2 représente un groupement alkyle linéaire ou ramifié en $C_1$ à $C_8$, hydroxyalkyle linéaire ou ramifié en $C_1$ à $C_8$ ou alcoxyalkyle en $C_1$ à $C_8$, des atomes d'oxygène pouvant être situés entre les atomes de carbone de la chaîne alkyle ; et R5 et R6 représentent indépendamment l'un de l'autre un hydrogène, un groupement alkyle linéaire ou ramifié en $C_1$ à $C_4$, hydroxyalkyle linéaire ou ramifié en $C_1$ à $C_4$, hydroxy, méthoxy, amino, dialkylamino ou un atome d'halogène ; et A représente un chlorure, un bromure, un iodure, un hydrogénosulfate, un monométhylsulfate, un sulfate, un hexafluorophosphate, un hexafluoroantimonate, un tétrafluoroborate, un tétraphénylborate.

3. Agent selon la revendication 2, **caractérisé en ce que** l'énamine des formules (II) à (IX) est choisie parmi la 3-éthyl-2-méthylènebenzothiazoline, la 2-méthylène-1,3,3-triméthylindoline, le chlorure de 1,2,3,3-tétraméthyl-3H-indolinium, le bromure de 1,2,3,3-tétraméthyl-3H-indolinium, l'iodure de 1,2,3,3-tétraméthyl-3H-indolinium, le sulfate de 1,2,3,3-tétraméthyl-3H-indolinium, l'hydrogénosulfate de 1,2,3,3-tétraméthyl-3H-indolinium, le méthylsulfate de 1,2,3,3-tétraméthyl-3H-indolinium, l'hexafluorophosphate de 1,2,3,3-tétraméthyl-3H-indolinium, l'hexafluoroantimonate de 1,2,3,3-tétraméthyl-3H-indolinium, le tétrafluoroborate de 1,2,3,3-tétraméthyl-3H-indolinium, la 5-chloro-2-méthylène-1,3,3-triméthyl-indoline ou ses sels, la 1-(2-hydroxyéthyl)-3,3-diméthyl-2-méthylèneindoline ou ses sels, le chlorure de 1,1,2,3-tétraméthyl-1H-benz[e]-indolinium, le bromure de 1,1,2,3-tétraméthyl-1H-benz[e]-indolinium, l'iodure de 1,1,2,3-tétraméthyl-1H-benz[e]-indolinium, le sulfate de 1,1,2,3-tétraméthyl-2H-benz[e]-indolinium, l'hexafluorophosphate de 1,1,2,3-tétraméthyl-1H-benz[e]-indolinium, le méthylsulfate de

1,1,2,3-tétraméthyl-2H-benz[e]-indolinium, l'hexafluoroantimonate de 1,1,2,3-tétraméthyl-1H-benz[e]-indolinium et le tétrafluoroborate de 1,1,2,3-tétraméthyl-1H-benz[e]-indolinium.

**4.** Agent selon l'une quelconque des revendications 1 à 3 , **caractérisé en ce que** le composé carbonyle est choisi parmi la vanilline (4-hydroxy-3-méthoxybenzaldéhyde), l'isovanilline (3-hydroxy-4-méthoxybenzaldéhyde), le 3,4-dihydroxybenzaldéhyde, le 4-hydroxybenzaldéhyde, le 3,5-diméthoxy-4-hydroxybenzaldéhyde, le 4-diméthylaminobenzaldéhyde, le 4-méthyl-5-imidazol-carboxaldéhyde, l'aldéhyde 4-diméthylaminocinnamique, le 4-hydroxy-2-méthoxybenzaldéhyde, le 3,5-diméthyl-4-hydroxybenzaldéhyde, le 4-diméthylamino-2-méthoxybenzaldéhyde, le 2-hydroxybenzaldéhyde, le 4-hydroxy-1-naphtaldéhyde, le 4-méthoxy-1-naphtaldéhyde, le 4-diméthylamino-1-naphtaldéhyde, le 4'-hydroxybiphényl-1-carbaldéhyde, le 2-hydroxy-3-méthoxybenzaldéhyde, le 2,4-dihydroxybenzaldéhyde, le 3,4-dihydroxybenzaldéhyde, le 2,5-dihydroxybenzaldéhyde, le 2,3,4-trihydroxybenzaldéhyde, le 3,4,5-trihydroxybenzaldéhyde, le 2,4,6-trihydroxybenzaldéhyde, le 2,4-diméthoxybenzaldéhyde, le 2,3-diméthoxybenzaldéhyde, le 2,5-diméthoxybenzaldéhyde, le 3,5-diméthoxybenzaldéhyde, le 3,4-diméthoxybenzaldéhyde, l'indol-3-carbaldéhyde, le benzène-1,4-dicarbaldéhyde, le 4-éthoxybenzaldéhyde, la 2-méthyl-1,4-naphtoquinone, le 4-carboxybenzaldéhyde, l'aldéhyde 4-hydroxy-3-méthoxycinnamique, l'aldéhyde 3,5-diméthoxy-4-hydroxycinnamique, le 3-méthoxy-4-(1-pyrrolidinyl)-benzaldéhyde, le 4-diéthylamino-3-méthoxybenzaldéhyde, le 1,2-phtaldialdéhyde, le pyrrol-2-aldéhyde, le thiophén-2-aldéhyde, le thiophén-3-aldéhyde, le chromone-3-carboxaldéhyde, le 6-méthyl-4-oxo-1(4H)-benzopyranne-3-carbaldéhyde, le N-méthylpyrrol-2-aldéhyde, le 5-méthylfurfural, le 6-hydroxychromén-3-carboxaldéhyde, le 6-méthylindol-3-carboxaldéhyde, le 4-dibutylaminobenzaldéhyde, le N-éthylcarbazol-3-aldéhyde, le 4-diéthylamino-2-hydroxybenzaldéhyde, le 3,4-diméthoxy-5-hydroxybenzaldéhyde, le 5-(4-(diéthylamino)phényl)-2,4-pentadiénal, le 2,3-thiophènedicarboxaldéhyde, le 2,5-thiophènedicarboxaldéhyde, le 2-méthoxy-1-naphtaldéhyde, le 3-éthoxy-4-hydroxybenzaldéhyde, le 2-nitrobenzaldéhyde, le 3-nitrobenzaldéhyde et le 4-nitrobenzaldéhyde.

**5.** Agent selon l'une quelconque des revendications 1 à 4, **caractérisé en ce que** l'énamine et le composé carbonyle sont contenus dans l'agent de teinture prêt à l'emploi à chaque fois en une quantité de 0,01 à 10 % en poids.

**6.** Agent selon l'une quelconque des revendications 1 à 5, **caractérisé en ce qu'**il présente un pH de 3 à 11.

**7.** Kit à plusieurs composants pour teindre et déteindre ultérieurement des fibres, **caractérisé en ce qu'**il contient une teinture A selon l'une quelconque des revendications 1 à 6 et un composant B pour déteindre, le composant B contenant au moins un sulfite.

**8.** Kit à plusieurs composants selon la revendication 7, **caractérisé en ce que** le sulfite est choisi parmi les sulfites d'ammonium, les sulfites alcalins et les sulfites alcalino-terreux.

**9.** Kit à plusieurs composants selon la revendication 7 ou 8, **caractérisé en ce que** le sulfite dans le composant B est contenu en une quantité totale de 0,1 à 10 % en poids.

**10.** Procédé pour la teinture temporaire de cheveux, dans lequel les cheveux sont teints avec un agent selon l'une quelconque des revendications 1 à 6 et sont à nouveau déteints à un moment ultérieur quelconque par traitement des cheveux avec une composition contenant un sulfite pendant une durée de 5 à 60 minutes à une température de 20 à 50°C.